# EUROPEAN PATENT APPLICATION

(11) **EP 1 495 768 A1**
(43) Date of publication of application: **12.01.2005**
(21) Application number: 03746168.8
(22) Date of filing: 10.04.2003
(51) Int. Cl.: A61K 45/00, A61K 31/519, A61P 5/24, A61P 15/12, A61P 43/00, C07D 495/04

(54) **PREVENTIVES/REMEDIES FOR HOTFLASH**

(30) Priority: 12.04.2002 JP 2002111235
(71) Applicant: Takeda Pharmaceutical Company Limited, Osaka-shi, Osaka 541-0045 (JP)
(72) Inventor: KUSAKA, Masami, Kobe-shi, Hyogo 651-2102 (JP); FURUYA, Shuichi, Tsukuba-shi, Ibaraki 305-0821 (JP)
(74) Representative: von Kreisler, Alek, Dipl.-Chem.
(86) International application number: PCT/JP2003/004554
(87) International publication number: WO 2003/086464

(57) **Abstract**

It is intended to provide a preventing or treating agent for hot flash which comprises a nonpeptidic compound having gonadotropin releasing hormone antagonistic activity, in particular, a preventing or treating agent for hot flash which comprises a nonpeptidic compound having gonadotropin releasing hormone antagonistic activity, wherein said compound is capable of entering the brain.

## Description

### Technical Field

The present invention relates to a preventing or treating agent for climacteric disorder, in particular, hot flash.

### Background Art

Females accounting for a half of humans enter menopause due to loss of the ovarian function and then are suffered from various symptoms referred to as climacteric disorder [vasomotor nervous disorder (hot flash, perspiration, palpitation, etc.), psychoneurotic disorder (excitement, insomnia, irritation, headache, etc.), atrophy of urogenital system, lipid metabolic disorder, osteoporosis, etc.] including hot flash (a local rise in the body surface temperature, and vertigo and glow accompanying it). Although a main cause for climacteric disorder is thought to be decrease in the sex hormone levels, it is not clear. Climacteric disorder leads women to reduction in QOL (Quality of Life) of their individual and social life. Thus, clarifying a cause of climacteric disorder and finding a treating method thereof are desired. In addition, sex hormone-dependent disease is treated by lowering the sex hormone level or inhibiting the sex hormone activity and such treatment produces climacteric disorder-like symptoms including hot flash. This is shown not only in women but also in men and this is a side effect of such treatment.

Although a cause of climacteric disorder is not clear, it is true that decrease in the sex hormone levels triggers it. Therefore, a sex hormone may be supplemented as a treatment for climacteric disorder. Such hormone replacement therapy shows a certain effect, but it has a problem of increase in carcinogenic risk. In addition, when treatment by lowering the sex hormone level or inhibiting the sex hormone activity is performed, supplementation of a sex hormone weakens the original drug efficacy, being not preferable.

Lowering the sex hormone level decreases the negative feedback of the sex hormone and promotes synthesis and secretion of GnRH (gonadotropin releasing hormone). Then, GnRH stimulates synthesis and secretion of LH and FSH to enhance their blood concentration. Therefore, various climacteric disorders including hot flash may be caused by increase in the GnRH, LH or FSH level.

Synthesis and secretion of GnRH and expression of a receptor for GnRH were confirmed not only in hypothalamus and pituitary gland but also in brain, but the function has not yet been clarified well. From an experiment of administration of GnRH or GnRH and an antagonist thereof into the brain of a rat, it was suggested that increasing GnRH level was involved in manifestation of hot flash and an antagonist of GnRH might suppress it. However, the antagonist used in the experiment was a peptidic antagonist (Brain Research 754 (1997) 88-94.) which was difficult to use clinically.

In addition, a method for suppressing hot flash accompanying prostate cancer treatment using PPI-149 which was a peptidic GnRH antagonist was reported (JP-A 2002-512976 (WO99/55358)).

Regarding a preventing or treating agent for climacteric disorder, in particular, hot flash, a medicament which is satisfactory to clinical use has not been reported yet. An object of the present invention is to provide a preventing or treating agent for climacteric disorder, in particular, hot flash. In the present invention, a preventing or treating agent includes an improving agent.

### Disclosure of Invention

The present inventors found out that non-peptidic compounds having gonadotropin releasing hormone antagonistic activity suppress action of intracerebral GnRH and thereby are effective as a preventing or treating agent for hot flash, and as a result of further study based on this finding, completed the present invention.

That is, the present invention relates to:
[1] a preventing or treating agent for hot flash which comprises a non-peptidic compound having gonadotropin releasing hormone antagonistic activity;
[2] the agent according to the above [1], wherein the compound is a compound capable of entering the brain,
[3] the agent according to the above [1], wherein the compound is a fused heterocyclic compound,
[4] the agent according to the above [1], wherein the compound is a compound represented by the formula: 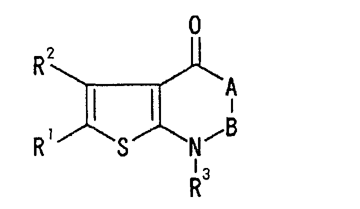 wherein R¹ represents (1) a hydrogen atom, (2) a group linking via a carbon atom, (3) a group linking via a nitrogen atom, (4) a group linking via an oxygen atom or (5) a group linking via a sulfur atom,
   R² represents (1) a hydrogen atom, (2) a group linking via a carbon atom, (3) a group linking via a nitrogen atom, (4) a group linking via an oxygen atom or (5) a group linking via a sulfur atom,
   R³ represents (1) a hydrogen atom, (2) alkyl or (3) -(CH₂)ₚQ (wherein p represents an integer of 0 to 3 and Q represents an optionally substituted homocyclic group or an optionally substituted heterocyclic group), 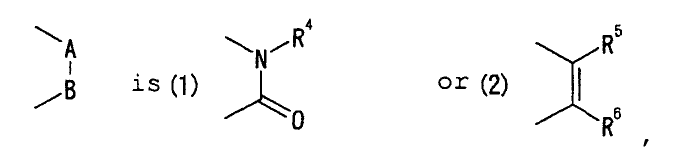 R⁴ represents (1) a hydrogen atom, (2) alkyl optionally substituted with alkoxy, (3) optionally substituted aryl, (4) optionally substituted aralkyl or (5) optionally substituted cycloalkyl,
   R⁵ represents (1) a hydrogen atom, (2) formyl, (3) cyano, (4) C₁₋₆alkyl optionally substituted with (i) a group linking via a sulfur atom or (ii) a group linking via an oxygen atom, (5) an optionally substituted heterocyclic group, (6) a group linking via a nitrogen atom, (7) a group linking via an oxygen atom, (8) a group linking via a sulfur atom, (9) optionally esterified, thioesterified or amidated carboxyl or (10) -C(O)R⁷ (wherein R⁷ represents an optionally substituted hydrocarbon group), and
   R⁶ represents (1) a hydrogen atom or (2) a group linking via a carbon atom, or a salt or prodrug thereof;
[5] the agent according to the above [4], wherein R¹ is optionally substituted C₆₋₁₄ aryl, R² is (1) C₁₋₃alkyl substituted with a group linking via a nitrogen atom or (2) a group linking via a nitrogen atom, R³ is -(CH₂)ₚQ (wherein p represents an integer of 0 to 3 and Q represents an optionally substituted homocyclic group or an optionally substituted heterocyclic group), 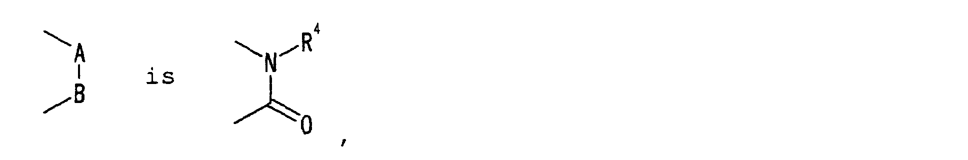 R⁴ is (1) C₁₋₆alkyl optionally substituted with C₁₋₆alkoxy or (2) optionally substituted C₆₋₁₄aryl;
[6] the agent according to the above [1], wherein the compound is a compound represented by the formula: 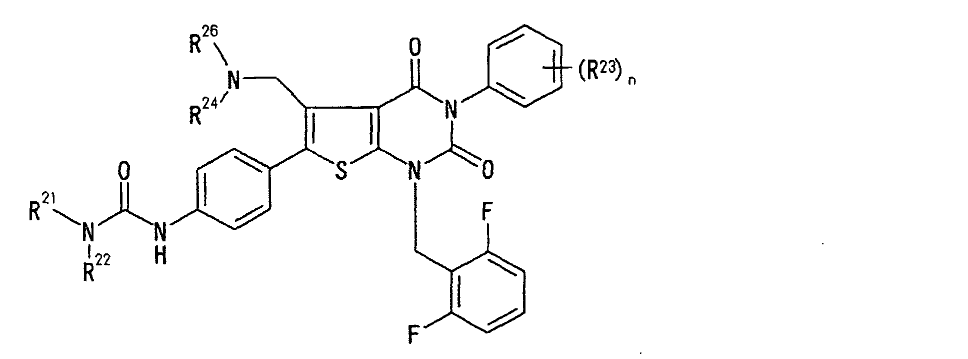 wherein R²¹ and R²² each represent (1) a hydrogen atom (2) hydroxy (3) C₁₋₄alkoxy, (4) C₁₋₄alkoxy-carbonyl or (5) optionally substituted C₁₋₄alkyl, R²³ represents (1) a hydrogen atom, (2) halogen, (3) hydroxy or (4) optionally substituted C₁₋₄alkoxy, or two R²³ adjacent to each other may be linked to form C₁₋₄ alkylenedioxy, R²⁴ represents (1) a hydrogen atom or (2) C₁₋₄alkyl, and R²⁶ represents (1) optionally substituted C₁₋₄alkyl or (2) a group represented by the formula: 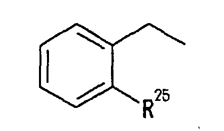 wherein R²⁵ represents a hydrogen atom or may be taken together with R²⁴ to form a heterocycle, and n represents an integer of 0 to 5, or a salt thereof;
[7] a method for preventing or treating hot flash, which comprises administering an effective amount of a non-peptidic compound having gonadotropin releasing hormone antagonistic activity to a mammal;
[8] use of a non-peptidic compound having gonadotropin releasing hormone antagonistic activity for preparation of a preventing or treating agent for hot flash; and the like.

The "non-peptidic compound having gonadotropin releasing hormone (GnRH) antagonistic activity" (GnRH antagonist) may be any non-peptidic compounds having gonadotropin releasing hormone antagonistic activity.

The non-peptidic compound having GnRH antagonistic activity may be, for example, a compound having a molecular weight of 1,000 or less, preferably a compound having a molecular weight of 900 or less, more preferably a compound having a molecular weight of 800 or less.

In addition, the compound preferably has good oral absorbability. For example, when 10 mg/kg of the compound is orally administered to a mammal, the compound exhibits preferably an absorption rate of 10% or larger, more preferably an absorption rate of 20% or larger.

In addition, the compound is preferably capable of entering the brain.

A particularly preferred example of the non-peptidic compound having GnRH antagonistic activity is a fused heterocyclic compound meeting the aforementioned conditions.

Such a fused heterocyclic compound includes a compound represented by the formula: 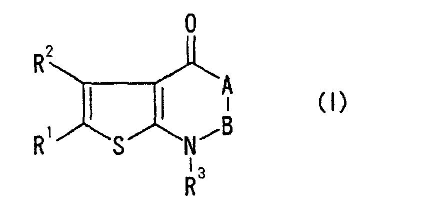 wherein R¹ represents (1) a hydrogen atom, (2) a group linking via a carbon atom, (3) a group linking via a nitrogen atom, (4) a group linking via an oxygen atom or (5) a group linking via a sulfur atom,
R² represents (1) a hydrogen atom, (2) a group linking via a carbon atom, (3) a group linking via a nitrogen atom, (4) a group linking via an oxygen atom or (5) a group linking via a sulfur atom,
R³ represents (1) a hydrogen atom, (2) alkyl or (3) -(CH₂)ₚQ (wherein p represents an integer of 0 to 3 and Q represents an optionally substituted homocyclic group or an optionally substituted heterocyclic group), 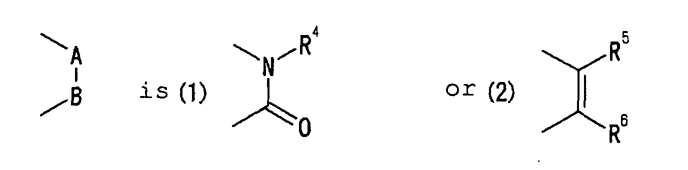 R⁴ represents (1) a hydrogen atom, (2) alkyl optionally substituted with alkoxy, (3) optionally substituted aryl, (4) optionally substituted aralkyl or (5) optionally substituted cycloalkyl,
R⁵ represents (1) a hydrogen atom, (2) formyl, (3) cyano, (4) C₁₋₆alkyl optionally substituted with (i) a group linking via a sulfur atom or (ii) a group linking via an oxygen atom, (5) an optionally substituted heterocyclic group, (6) a group linking via a nitrogen atom, (7) a group linking via an oxygen atom, (8) a group linking via a sulfur atom, (9) optionally esterified, thioesterified or amidated carboxyl or (10) -C(O)R⁷ (wherein R⁷ represents an optionally substituted hydrocarbon group), and
R⁶ represents (1) a hydrogen atom or (2) a group linking via a carbon atom, (hereinafter, abbreviated as Compound (I) in some cases) or a salt or prodrug thereof.
A definition of each substituent in the Compound (I) is shown below.

The "group linking via a carbon atom" represented by R¹, R² or R⁶ includes (1) optionally substituted alkyl, (2) optionally substituted cycloalkyl, (3) optionally substituted alkenyl, (4) optionally substituted aryl, (5) optionally substituted aralkyl, (6) a heterocyclic group linking via a carbon atom (said heterocyclic group may be substituted), (7) formyl, (8) optionally esterfied or amidated carboxyl, (9) cyano and (10) amidino.

The alkyl of the "optionally substituted alkyl" in the definition of the "group linking via a carbon atom" represented by R¹, R² or R⁶ includes straight and branched C₁₋₆ alkyl such as methyl, ethyl, propyl, isopropyl, butyl, isobutyl, sec-butyl, tert-butyl, pentyl, isopentyl, neopentyl, hexyl, isohexyl, 3-methylpentyl, neohexyl and 2,3-dimethylbutyl.

The substituent of the "optionally substituted alkyl" includes (1) C₆₋₁₄ aryl (e.g. phenyl, naphthyl, etc.) optionally substituted with 1 to 4 substituents selected from (i) hydroxyl, (ii) amino, (iii) mono-or di-C₁₋₆ alkylamino (e.g. methylamino, ethylamino, propylamino, dimethylamino, diethylamino, etc.), (iv) C₁₋₆ alkoxy (e.g. methoxy, ethoxy, propoxy, butoxy, pentoxy, hexyloxy, etc.) and (v) halogen (e.g. fluorine, chlorine, bromine, iodine), (2) hydroxyl, (3) carboxy, (4) nitro, (5) C₁₋₆ alkoxy (e.g. methoxy, ethoxy, propoxy, isopropoxy, butoxy, pentoxy, hexyloxy, etc.), (6) C₁₋₆ alkyl-carbonyloxy (e.g. acetoxy, propionyloxy, butyryloxy, isobutyryloxy, valeryloxy, isovaleryloxy, pivaloyloxy, pentylcarbonyloxy, hexylcarbonyloxy, etc.), (7) C₁₋₆ alkylthio (e.g. methylthio, ethylthio, propylthio, isopropylthio, butylthio, isobutylthio, sec-butylthio, tert-butylthio, pentylthio, hexylthio, etc.), (8) C₁₋₆alkylsulfinyl (e.g. methylsulfinyl, ethylsulfinyl, propylsulfinyl, isopropylsulfinyl, butylsulfinyl, isobutylsulfinyl, sec-butylsulfinyl, tert-butylsulfinyl, pentylsulfinyl, hexylsulfinyl, etc.), (9) C₁₋₆alkylsulfonyl (e.g. methylsulfonyl, ethylsulfonyl, propylsulfonyl, isopropylsulfonyl, butylsulfonyl, isobutylsulfonyl, sec-butylsulfonyl, tert-butylsulfonyl, pentylsulfonyl, hexylsulfonyl, etc.), (10) halogen (e.g. fluorine, chlorine, bromine, iodine), (11) a group linking via a nitrogen atom and (12) a heterocyclic group.

The "group linking via a nitrogen atom" as the substituent of the "optionally substituted alkyl" includes (1) -NR⁸R⁹ (wherein R⁸ represents a hydrogen atom, optionally substituted C₁₋₆alkyl, optionally substituted C₃₋₆ cycloalkyl, optionally substituted C₆₋₁₄ aryl, optionally substituted C₇₋₂₀ aralkyl, acyl, optionally substituted carbamoyl or heterocyclic group, and R⁹ represents a hydrogen atom or optionally substituted C₁₋₆alkyl), and (2) a heterocyclic group linking via a nitrogen atom (e.g. 1H-1-pyrrolyl, 1-imidazolyl, pyrazolyl, indolyl, 1H-1-indazolyl, 7-purinyl, 1-pyrrolidinyl, 1-pyrrolinyl, 1-imidazolidinyl, pyrazolidinyl, piperazinyl, pyrazolinyl, 1-piperidinyl, 4-morpholinyl, 4-thiomorpholinyl, 2-isoindolyl, 2-(1,2,3,4-tetrahydro)isoquinolyl, etc.).

The C₁₋₆alkyl of the "optionally substituted C₁₋₆ alkyl" represented by R⁸ or R⁹ includes methyl, ethyl, propyl, isopropyl, butyl, isobutyl, sec-butyl, tert-butyl, pentyl, isopentyl, neopentyl, hexyl, isohexyl, 3-methylpentyl, neohexyl, and 2,3-dimethylbutyl.

The substituent of the "optionally substituted C₁₋₆ alkyl" represented by R⁸ or R⁹ includes (1) C₁₋₆ alkyl (e.g. methyl, ethyl, propyl, isopropyl, butyl, isobutyl, sec-butyl, tert-butyl, pentyl, isopentyl, neopentyl, hexyl, isohexyl, 3-methylpentyl, neohexyl, 2,3-dimethylbutyl etc.), (2) C₂₋₆ alkenyl (e.g. vinyl, 1-methylvinyl, 1-propenyl, allyl etc.), (3) C₂₋₆ alkynyl (e.g. ethynyl, 1-propynyl, propargyl etc.), (4) C₃₋₆ cycloalkyl (e.g. cyclopropyl, cyclobutyl, cyclopentyl, cyclohexyl etc.), (5) C₅₋₇ cycloalkenyl (e.g. cyclopentenyl, cyclohexenyl etc.), (6) C₇₋₁₁ aralkyl (e.g. benzyl, α-methylbenzyl, phenethyl etc.), (7) C₆₋₁₄ aryl (e.g. phenyl, naphthyl etc.), (8) C₁₋₆ alkoxy (e.g. methoxy, ethoxy, propoxy, isopropoxy, butoxy, isobutoxy, sec-butoxy, tert-butoxy etc.), (9) C₆₋₁₄ aryloxy (e.g. phenoxy, 1-naphthoxy, 2-naphthoxy etc.), (10) C₁₋₆ alkanoyl (e.g. formyl, acetyl, propionyl, butyryl, isobutyryl etc.), (11) C₆₋₁₄ aryl-carbonyl (e.g. benzoyl, 1-naphtylcarbonyl, 2-naphtylcarbonyl etc.), (12) C₁₋₆ alkanoyloxy (e.g. formyloxy, acetoxy, propionyloxy, butyryloxy, isobutyryloxy, etc.), (13) C₆₋₁₄ arylcarbonyloxy (e.g. benzoyloxy, 1-naphtylcarbonyloxy, 2-naphtylcarbonyloxy etc.), (14) carboxy, (15) C₁₋₆alkoxycarbonyl (e.g. methoxycarbonyl, ethoxycarbonyl, propoxycarbonyl, isopropoxycarbonyl, butoxycarbonyl, isobutoxycarbonyl, tert-butoxycarbonyl etc.), (16) carbamoyl, (17) N-mono-C₁₋₄ alkylcarbamoyl (e.g. N-methylcarbamoyl, N-ethylcarbamoyl, N-propylcarbamoyl, N-isopropylcarbamoyl, N-butylcarbamoyl etc.), (18) N,N-di-C₁₋₄ alkylcarbamoyl (e.g. N,N-dimethylcarbamoyl, N,N-diethylcarbamoyl, N,N-dipropylcarbamoyl, N,N-dibutylcarbamoyl etc.), (19) cyclic aminocarbonyl (e.g. 1-aziridinylcarbonyl, 1-azetidinylcarbonyl, 1-pyrrolidinylcarbonyl, 1-piperidinylcarbonyl, N-methylpiperazinylcarbonyl, morpholinocarbonyl etc.), (20) halogen (e.g. fluorine, chlorine, bromine, iodine), (21) C₁₋₄ alkyl substituted with 1 to 3 halogen (e.g. chloromethyl, dichloromethyl, trifluoromethyl, trifluoroethyl etc.), (22) oxo, (23) amidino, (24) imino, (25) amino, (26) mono- or di-C₁₋₄ alkylamino (e.g. methylamino, ethylamino, propylamino, isopropylamino, butylamino, isobutylamino, sec-butylamino, tert-butylamino, pentylamino, hexylamino, dimethylamino, diethylamino, dipropylamino etc.), (27) 3- to 6-membered cyclic amino optionally containing 1 to 3 heteroatoms selected from an oxygen atom, a sulfur atom and a nitrogen atom in addition to carbon atoms and a nitrogen atom (e.g. aziridinyl, azetidinyl, pyrrolidinyl, pyrrolinyl, pyrrolyl, imidazolyl, pyrazolyl, imidazolidinyl, piperidino, morpholino, dihydropyridyl, pyridyl, N-methylpiperazinyl, N-ethylpiperazinyl etc.), (28) C₁₋₆alkanoylamino (e.g. formylamino, acetylamino, trifluoroacetylamino, propionylamino, butyrylamino, isobutyrylamino etc.), (29) benzamide, (30) carbamoylamino, (31) (N-C₁₋₄ alkylcarbamoyl)amino (e.g. (N-methylcarbamoyl)amino, (N-ethylcarbamoyl) amino, (N-propylcarbamoyl) amino, (N-isopropylcarbamoyl) amino, (N-butylcarbamoyl)amino etc.), (32) (N, N-di-C₁₋₄ alkylcarbamoyl) amino (e.g. (N,N-dimethylcarbamoyl) amino, (N,N-diethylcarbamoyl)amino, (N,N-dipropylcarbamoyl) amino, (N,N-dibutylcarbamoyl)amino etc.), (33) C₁₋₆alkylenedioxy (e.g. -OCH₂O-, -O(CH₂)₂O-, -O(CH₂)₃O-, -O(CH₂)₄O-, -O(CH₂)₅O-, -O(CH₂)₆O- etc.), (34) dihydroboryl, (35) hydroxy, (36) epoxy, (37) nitro, (38) cyano, (39) mercapto, (40) sulfo, (41) sulfino, (42) phosphono, (43) sulfamoyl, (44) N-C₁₋₆ alkylsufamoyl (e.g. N-methylsulfamoyl, N-ethylsulfamoyl, N-propylsufamoyl, N-isopropylsulfamoyl, N-butylsufamoyl etc.), (45) N,N-diC₁₋₆alkylsulfamoyl (e.g. N,N-dimethylsulfamoyl, N,N-diethylsulfamoyl, N,N-dipropylsulfamoyl, N,N-dibutylsulfamoyl etc.), (46) C₁₋₆ alkylthio (e.g. methylthio, ethylthio, propylthio, isopropylthio, butylthio, sec-butylthio, tert-butylthio etc.), (47) phenylthio, (48) C₁₋₆alkylsulfinyl (e.g. methylsulfinyl, ethylsulfinyl, propylsulfinyl, butylsulfinyl etc.), (49) phenylsulfinyl, (50) C₁₋₆ alkylsulfonyl (e.g. methylsulfonyl, ethylsulfonyl, propylsulfonyl, butylsulfonyl etc.), and (51) phenylsulfonyl. The optionally substituted C₁₋₆alkyl may have 1 to 6, preferably 1 to 3 substituents selected from the above-mentioned substituents at substitutable positions.

The C₃₋₆cycloalkyl of the "optionally substituted C₃₋₆ cycloalkyl" represented by R⁸ includes cyclopropyl, cyclobutyl, cyclopentyl, and cyclohexyl.

The substituent of the "optionally substituted C₃₋₆ cycloalkyl" represented by R⁸ includes the same substituents as those of the "optionally substituted C₁₋₆ alkyl" represented by R⁸ or R⁹ mentioned above, and the optionally substituted C₃₋₆ cycloalkyl may have 1 to 6, preferably 1 to 3 substituents at substitutable positions.

The C₆₋₁₄ aryl of the "optionally substituted C₆₋₁₄ aryl" represented by R⁸ includes phenyl, naphthyl and anthracenyl.

The substituent of the "optionally substituted C₆₋₁₄ aryl" represented by R⁸ includes the same substituents as those of the "optionally substituted C₁₋₆ alkyl" represented by R⁸ or R⁹ mentioned above excluding oxo and epoxy, and the optionally substituted C₆₋₁₄ aryl may have 1 to 6, preferably 1 to 3 substituents at substitutable positions.

The C₇₋₂₀ aralkyl of the "optionally substituted C₇₋₂₀ aralkyl" represented by R⁸ includes benzyl, phenethyl, phenylpropyl, benzhydryl and trityl.

The substituent of the "optionally substituted C₇₋₂₀ aralkyl" represented by R⁸ includes the same substituents as those of the "optionally substituted C₁₋₆ alkyl" represented by R⁸ or R⁹ mentioned above, and the optionally substituted C₇₋₂₀ aralkyl may have 1 to 6, preferably 1 to 3 substituents at substitutable positions.

The "acyl" represented by R⁸ includes groups formed by linking the "optionally substituted C₁₋₆ alkyl", the "optionally substituted C₃₋₆ cycloalkyl", the "optionally substituted C₆₋₁₄ aryl" or the "optionally substituted C₇₋₂₀ aralkyl" represented by R⁸ with carbonyl, sulfinyl or sulfonyl.

The substituent of the "optionally substituted carbamoyl" represented by R⁸ includes (1) optionally substituted C₁₋₆ alkyl, (2) optionally substituted C₃₋₆ cycloalkyl, (3) optionally substituted C₆₋₁₄ aryl, (4) optionally substituted C₇₋₂₀ aralkyl, (5) hydroxy, (6) optionally substituted C₁₋₆ alkoxy and (7) optionally substituted C₁₋₆ alkoxy-carbonyl, and the optionally substituted carbamoyl may have 1 or 2 substituents selected from these substituents.

Examples of the "optionally substituted C₁₋₆ alkyl" as the substituent of the "optionally substituted carbamoyl" represented by R⁸ are the same as those of the "optionally substituted C₁₋₆ alkyl" represented by R⁸ or R⁹ mentioned above.

Examples of the "optionally substituted C₃₋₆ cycloalkyl", the "optionally substituted C₆₋₁₄ aryl" and the "optionally substituted C₇₋₂₀ aralkyl" as the substituent of the "optionally substituted carbamoyl" represented by R⁸ are the same as those of the "optionally substituted C₃₋₆ cycloalkyl", the "optionally substituted C₆₋₁₄ aryl" and the "optionally substituted C₇₋₂₀ aralkyl" represented by R⁸ mentioned above, respectively.

The C₁₋₆ alkoxy of the "optionally substituted C₁₋₆ alkoxy" as the substituent of the "optionally substituted carbamoyl" represented by R⁸ includes methoxy, ethoxy, propoxy, isopropoxy, butoxy, isobutoxy, sec-butoxy, tert-butoxy, pentoxy, and hexyloxy.

The substituent of the "optionally substituted C₁₋₆ alkoxy" includes the same substituents as those of the "optionally substituted C₁₋₆alkyl" represented by R⁸ mentioned above, and the optionally substituted C₁₋₆ alkoxy may have 1 to 6, preferably 1 to 3 substituents at substitutable positions.

The "optionally substituted C₁₋₆ alkoxy-carbonyl" as the substituent of the "optionally substituted carbamoyl" represented by R⁸ includes groups formed by linking the "optionally substituted C₁₋₆ alkoxy" as the substituent of the "optionally substituted carbamoyl" represented by R⁸ mentioned above with carbonyl.

The "heterocyclic group" represented by R⁸ includes (1) a 5-membered cyclic group containing 1 to 4 heteroatoms selected from an oxygen atom, a sulfur atom and a nitrogen atom in addition to carbon atoms (e.g. 2-thienyl, 3-thienyl, 2-furyl, 3-furyl, 2-pyrrolyl, 3-pyrrolyl, 2-oxazolyl, 4-oxazolyl, 5-oxazolyl, 2-thiazolyl, 4-thiazolyl, 5-thiazolyl, 3-pyrazolyl, 4-pyrazolyl, 5-pyrazolyl, 2-imidazolyl, 4-imidazolyl, 5-imidazolyl, 3-isoxazolyl, 6-isoxazolyl, 5-isoxazolyl, 3-isothiazolyl, 4-isothiazolyl, 5-isothiazolyl, 3-(1,2,4-oxadiazolyl), 5-(1,2,4-oxadiazolyl), 1,3,4-oxadiazolyl, 3-(1,2,4-thiadiazolyl), 5-(1,2,4-thiadiazolyl), 1,3,4-thiadiazolyl, 4-(1,2,3-thiadiazolyl), 5-(1,2,3-thiadiazolyl), 1,2,5-thiadiazolyl, 1,2,3-triazolyl, 1,2,4-triazolyl, 1H-tetrazolyl, 2H-tetrazolyl, oxoimidazinyl, dioxotriazinyl, pyrrolidinyl etc.), (2) a 6-membered cyclic group containing 1 to 4 heteroatoms selected from an oxygen atom, a sulfur atom and a nitrogen atom in addition to carbon atoms (e.g. 2-pyridyl, 3-pyridyl, 4-pyridyl, N-oxide-2-pyridyl, N-oxide-3-pyridyl, N-oxide-4-pyridyl, 2-pyrimidinyl, 4-pyrimidinyl, 5-pyrimidinyl, N-oxide-2-pyrimidinyl, N-oxide-4-pyrimidinyl, N-oxide-5-pyrimidinyl, 2-thiomorpholinyl, 3-thiomorpholinyl, 2-morpholinyl, 3-morpholinyl, piperidinyl, pyranyl, thiopyranyl, 1,4-oxazinyl, 1,4-thiazinyl, 1,3-thiazinyl, 2-piperazinyl, 3-piperazinyl, triazinyl, oxotriazinyl, 3-pyridazinyl, 4-pyridazinyl, pyrazinyl, N-oxide-3-pyridazinyl, N-oxide-4-pyridazinyl etc.), and (3) a bicyclic or tricyclic fused cyclic group containing 1 to 4 heteroatoms selected from an oxygen atom, a sulfur atom and a nitrogen atom in addition to carbon atoms (e.g. benzofuryl, benzothiazolyl, benzoxazolyl, tetrazolo[1,5-b]pyridazinyl, triazolo[4,5-b]pyridazinyl, benzimidazolyl, quinolyl, isoquinolyl, cinnolyl, phthalazinyl, quinazolinyl, quinoxalinyl, indolizinyl, quinolizinyl, 1,8-naphthyridinyl, purinyl, pteridinyl, dibenzofuranyl, carbazolyl, acridinyl, phenanthridinyl, chromanyl, benzoxazinyl, phenazinyl, phenothiazinyl, phenoxazinyl etc.).

Examples of the heterocyclic group as the substituent of the "optionally substituted alkyl" in the definition of the "group linking via a carbon atom" represented by R¹, R² or R⁶ are the same as those of the "heterocyclic group" represented by R⁸ mentioned above.

The cycloalkyl of the "optionally substituted cycloalkyl" in the definition of the "group linking via a carbon atom" represented by R¹, R² or R⁶ includes C₃₋₆ cycloalkyl such as cyclopropyl, cyclobutyl, cyclopentyl and cyclohexyl.

The substituent of the "optionally substituted cycloalkyl" includes the same substituents as those of the "optionally substituted alkyl" in the definition of the "group linking via a carbon atom" represented by R¹, R² or R⁶, and the optionally substituted cycloalkyl may have 1 to 6, preferably 1 to 3 substituents at substitutable positions.

The alkenyl of the "optionally substituted alkenyl" in the definition of the "group linking via a carbon atom" represented by R¹, R² or R⁶ includes C₂₋₆ alkenyl such as vinyl, butadienyl and hexatrienyl.

The substituent of the "optionally substituted alkenyl" includes the same substituents as those of the "optionally substituted alkyl" in the definition of the "group linking via a carbon atom" represented by R¹, R² or R⁶, and the optionally substituted alkenyl may have 1 to 6, preferably 1 to 3 substituents at substitutable positions.

The aryl of the "optionally substituted aryl" in the definition of the "group linking via a carbon atom" represented by R¹, R² or R⁶ includes C₆₋₁₄ aryl such as phenyl, naphthyl and anthracenyl.

The substituent of the "optionally substituted aryl" includes the same substituents as those of the "optionally substituted alkyl" in the definition of the "group linking via a carbon atom" represented by R¹, R² or R⁶, such as C₁₋₆ alkoxycarbonyl (e.g. methoxycarbonyl, ethoxycarbonyl, propoxycarbonyl, isopropoxycarbonyl, butoxycarbonyl, isobutoxycarbobnyl, sec-butoxycarbonyl, tert-butoxycarbonyl, pentyloxycarbonyl, hexyloxycarbonyl etc.), carbamoyl, and N-mono-C₁₋₆alkylcarbamoyl (e.g. N-methylcarbamoyl, N-ethylcarbamoyl, N-propylcarbamoyl, N-isopropylcarbamoyl etc.), N,N-di-C₁₋₆alkylcarbamoyl (e.g. N,N-dimethylcarbamoyl, N,N-diethylcarbamoyl, N,N-dipropylcarbamoyl etc.), and the optionally substituted aryl may have 1 to 6, preferably 1 to 3 substituents at substitutable positions.

The aralkyl of the "optionally substituted aralkyl" in the definition of the "group linking via a carbon atom" represented by R¹, R² or R⁶ includes C₇₋₂₀ aralkyl such as benzyl, benzhydryl and trityl.

The substituent of the "optionally substituted aralkyl" includes the same substituents as those of the "optionally substituted alkyl" in the definition of the "group linking via a carbon atom" represented by R¹, R² or R⁶, and the optionally substituted aralkyl may have 1 to 6, preferably 1 to 3 substituents at substitutable positions.

Examples of the "heterocyclic group linking via a carbon atom" in the definition of the "group linking via a carbon atom" represented by R¹, R² or R⁶ are the same as those of the heterocyclic group represented by R⁸.

The "heterocyclic group linking via a carbon atom" may be substituted and the substituent includes the same substituents as those of the "optionally substituted alkyl" in the definition of the "group linking via a carbon atom" represented by R¹, R² or R⁶. The heterocyclic group linking via a carbon atom may have 1 to 6, preferably 1 to 3 substituents at substitutable positions.

The "optionally esterified carboxyl" in the definition of the "group linking via a carbon atom" represented by R¹, R² or R⁶ includes a group represented by -CO₂R¹⁰, wherein R¹⁰ represents hydrogen, optionally substituted alkyl, optionally substituted cycloalkyl, optionally substituted aryl, optionally substituted aralkyl or a heterocyclic group linking via a carbon atom (said heterocyclic group may be substituted).

Examples of the "optionally substituted alkyl", the "optionally substituted cycloalkyl", the "optionally substituted aryl", the "optionally substituted aralkyl" and the "heterocyclic group linking via a carbon atom (said heterocyclic group may be substituted)" represented by R¹⁰ are the same as those of the "optionally substituted alkyl", the "optionally substituted cycloalkyl", the "optionally substituted aryl", the "optionally substituted aralkyl" and the "heterocyclic group linking via a carbon atom (said heterocyclic group may be substituted)" as the "group linking via a carbon atom" represented by R¹, R² or R⁶, respectively.

The "optionally amidated carboxyl" in the definition of the "group linking via a carbon atom" represented by R¹, R² or R⁶ includes a group represented by -CONR⁸R⁹, wherein R⁸ and R⁹ are as defined above.

Examples of the "group linking via a nitrogen atom" represented by R¹, R² or R⁵ are the same as those of the "group linking via a nitrogen atom" as the substituent of the "optionally substituted alkyl" in the definition of the "group linking via a carbon atom" represented by R¹, R² or R⁶.

The "group linking via an oxygen atom" represented by R¹, R² or R⁵ includes a group represented by -OR¹¹, wherein R¹¹ represents optionally substituted C₁₋₆ alkyl, optionally substituted C₃₋₆ cycloalkyl, optionally substituted C₆₋₁₄ aryl, optionally substituted C₇₋₂₀ aralykl or an optionally substituted heterocyclic group.

Examples of the "optionally substituted C₁₋₆ alkyl" represented by R¹¹ are the same as those of the "optionally substituted C₁₋₆alkyl" represented by R⁸ or R⁹ mentioned above.

Examples of the "optionally substituted C₃₋₆ cycloalkyl", the "optionally substituted C₆₋₁₄ aryl", the "optionally substituted C₇₋₂₀ aralkyl" and the "optionally substituted heterocyclic group" represented by R¹¹ are the same as those of the "optionally substituted C₃₋₆ cycloalkyl", the "optionally substituted C₆₋₁₄ aryl", the "optionally substituted C₇₋₂₀ aralkyl" and the "optionally substituted heterocyclic group" represented by R⁸ mentioned above.

The "group linking via a sulfur atom" represented by R¹, R² or R⁵ includes a group represented by -SR¹¹, wherein R¹¹ is as defined above.

The alkyl represented by R³ includes C₁₋₆ alkyl such as methyl, ethyl, propyl, isopropyl, butyl, isobutyl, sec-butyl, tert-butyl, pentyl and hexyl.

The "optionally substituted homocyclic group" represented by Q includes (1) optionally substituted aryl and (2) optionally substituted cycloalkyl.

The aryl of the "optionally substituted aryl" in the definition of the "optionally substituted homocyclic group" represented by Q includes C₆₋₁₄ aryl such as phenyl, 1-naphthyl, 2-naphthyl, anthryl, phenanthryl and acenaphthylenyl.

The substituent of the "optionally substituted aryl" in the definition of the "optionally substituted homocyclic group" represented by Q includes (i) C₁₋₆ alkyl (e.g. methyl, ethyl, propyl, isopropyl, butyl, isobutyl, sec-butyl, tert-butyl, pentyl, hexyl etc.), (ii) C₂₋₆alkenyl (e.g. vinyl, allyl, 1-butenyl, 2-butenyl etc.), (iii) C₂₋₆ alkynyl (e.g. ethynyl, propargyl, 2-butynyl, 5-hexynyl etc.), (iv) C₃₋₆ cycloalkyl (e.g. cyclopropyl, cyclobutyl, cyclopentyl, cyclohexyl etc.), (v) C₆₋₁₄ aryl (e.g. phenyl, 1-naphthyl, 2-naphthyl etc.), (vi) C₇₋₁₄ aralkyl (e.g. benzyl, phenethyl etc.), (vii) nitro, (viii) hydroxyl, (ix) mercapto, (x) cyano, (xi) carbamoyl, (xii) carboxyl, (xiii) C₁₋₆ alkoxycarbonyl (e.g. methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, hexyloxycarbonyl etc.), (xiv) sulfo, (xv) halogen (e.g. fluorine, chlorine, bromine, iodine), (xvi) C₁₋₆ alkoxy (e.g, methoxy, ethoxy, propoxy, isopropoxy, butoxy, isobutoxy, sec-butoxy, tert-butoxy, pentyloxy, hexyloxy etc.) optionally substituted with C₁₋₆ alkoxy (e.g. methoxy, ethoxy, propoxy, isopropoxy, butoxy, isobutoxy, sec-butoxy, tert-butoxy, pentyloxy, hexyloxy etc.), (xvii) C₆₋₁₀ aryloxy (e.g. phenoxy, 1-naphthyloxy, 2-naphthyloxy etc.), (xviii) C₁₋₆ alkylthio (e.g. methylthio, ethylthio, propylthio, isopropyothio, butylthio, isobutylthio, sec-butylthio, tert-butylthio, pentylthio, hexylthio etc.), (xix) C₆₋₁₀ arylthio (e.g. phenylthio, 1-naphthylthio, 2-naphthylthio etc.), (xx) C₁₋₆ alkylsulfinyl (e.g. methylsulfinyl, ethylsulfinyl, propylsulfinyl, isopropylsulfinyl, butylsulfinyl, isobutylsulfinyl, sec-butylsulfinyl, tert-butylsulfinyl, pentylsulfinyl, hexylsulfinyl etc.), (xxi) C₆₋₁₀ arylsulfinyl (e.g. phenylsulfinyl, 1-naphthylsulfinyl, 2-naphthylsulfinyl etc), (xxii) C₁₋₆ alkylsulfonyl, (e.g. methylsulfonyl, ethylsulfonyl, propylsulfonyl, isopropylsulfonyl, butylsulfonyl, isobutylsulfonyl, sec-butylsulfonyl, tert-butylsulfonyl, pentylsulfonyl, hexylsulfonyl etc.), (xxiii) C₆₋₁₀ arylsulfonyl (e.g. phenylsulfonyl, 1-naphthylsulfonyl, 2-naphthylsulfonyl etc.), (xxiv) amino, (xxv) C₁₋₆acylamino (e.g. formylamino, acetylamino, propionylamino, butyrylamino, isobutyrylamino, valerylamino etc.), (xxvi) mono-C₁₋₆ alkylamino (e.g. methylamino, ethylamino, propylamino, isopropylamino, butylamino etc.), (xxvii) di-C₁₋₆alkylamino (e.g. dimethylamino, diethylamino, dipropylamino, diisopropylamino, dibutylamino etc.), (xxviii) C₃₋₆ cycloalkylamino (e.g. cyclopropylamino, cyclobutylamino, cyclopentylamino, cyclohexylamino etc.), (xxix) C₆₋₁₀ arylamino (e.g. anilino, 1-naphthylamino, 2-naphthylamino etc.), (xxx) C₁₋₆ acyl (e.g. formyl, acetyl, propionyl, butyryl, isobutyryl, valeryl etc.), (xxxi) C₆₋₁₀ arylcarbonyl (e.g. benzoyl, 1-naphthylcarbonyl, 2-naphthylcarbonyl etc.), and (xxxii) C₁₋₄ alkylenedioxy (e.g. -OCH₂O-, -(CH₂)₂O-, -O(CH₂)₃O-, -O(CH₂)₄O-, (xxxiii) a 5- or 6-membered heterocyclic group containing 1 to 4 heteroatoms selected from an oxygen atom, a sulfur atom and a nitrogen atom in addition to carbon atoms (e.g. 2-thienyl, 3-thienyl, 2-furyl, 3-furyl, 2-pyrrolyl, 3-pyrrolyl, 2-oxazolyl, 4-oxazolyl, 5-oxazolyl, 2-thiazolyl, 4-thiazolyl, 5-thiazolyl, 3-pyrazolyl, 4-pyrazolyl, 5-pyrazolyl, 2-imidazolyl, 4-imidazolyl, 5-imidazolyl, 3-isoxazolyl, 4-isoxazolyl, 5-isoxazolyl, 3-isothiazolyl, 4-isothiazolyl, 5-isothiazolyl, 3-(1,2,4-oxadiazolyl) , 5-(1,2,4-oxadiazolyl), 1,3,4-oxadiazolyl, 3-(1,2,4-thiadiazolyl), 5-(1,2,4-thiadiazolyl), 1,3,4-thiadiazolyl, 4-(1,2,3-thiadiazolyl), 5-(1,2,3-thiadiazolyl), 1,2,5-thiadiazolyl, 1,2,3-triazolyl, 1,2,4-triazolyl, 1H-tetrazolyl, 2H-tetrazolyl, oxoimidazinyl, dioxotriazinyl, pyrrolidinyl, 2-pyridyl, 3-pyridyl, 4-pyridyl, 2-pyrimidinyl, 4-pyrimidinyl, 5-pyrimidinyl, 2-thiomorpholinyl, 3-thiomorpholinyl, 2-morpholinyl, 3-morpholinyl, piperidinyl, pyranyl, thiopyranyl, 1,4-oxazinyl, 1,4-thiazinyl, 1,3-thiazinyl, 2-piperazinyl, 3-piperazinyl, triazinyl, oxotriazinyl, 3-pyridazinyl, 4-pyridazinyl, pyrazinyl etc.), and the optionally substituted aryl may have 1 to 6, preferably 1 to 3 substituents at substitutable positions.

The cycloalkyl of the "optionally substituted cycloalkyl" in the definition of the "optionally substituted homocyclic group" represented by Q includes C₃₋₆ cycloalkyl such as cyclopropyl, cyclobutyl, cyclopentyl and cyclohexyl.

The substituent of the "optionally substituted cycloalkyl" in the definition of the "optionally substituted homocyclic group" represented by Q includes oxo, thioxo, and the same substituents as those of the "optionally substituted aryl" in the definition of the "optionally substituted homocyclic group" represented by Q, and the optionally substituted cycloalkyl may have 1 to 6, preferably 1 to 3 substituents at substitutable positions.

Examples of the heterocyclic group of the "optionally substituted heterocyclic group" represented by Q are the same as those of the "heterocyclic group" represented by R⁸.

The substituent of the "optionally substituted heterocyclic group" represented by Q includes the same substituents as those of the "optionally substituted aryl" in the definition of the "optionally substituted homocyclic group" represented by Q, and the optionally substituted heterocyclic group may have 1 to 6, preferably 1 to 3 substituents at substitutable positions.

The "alkyl" of the "alkyl optionally substituted with alkoxy" represented by R⁴ includes C₁₋₆ alkyl such as methyl, ethyl, propyl, isopropyl, butyl, isobutyl, sec-butyl, tert-butyl, pentyl, isopentyl, neopentyl, hexyl, isohexyl, 3-methylpentyl, neohexyl and 2,3-dimethylbutyl.

The "alkoxy" in the "alkyl optionally substituted with alkoxy" represented by R⁴ includes C₁₋₆ alkoxy such as methoxy, ethoxy, propoxy, isopropoxy, butoxy, isobutoxy, sec-butoxy, tert-butoxy, pentyloxy and hexyloxy.

The "aryl" of the "optionally substituted aryl" represented by R⁴ includes C₆₋₁₄ aryl such as phenyl, 1-naphthyl and 2-naphthyl.

The substituent of the "optionally substituted aryl" represented by R⁴ includes the same substituents as those of the "optionally substituted aryl" in the definition of the "optionally substituted homocyclic group" represented by Q, and the optionally substituted aryl may have 1 to 6, preferably 1 to 3 substituents at substitutable positions.

The aralkyl of the "optionally substituted aralkyl" represented by R⁴ includes C₇₋₂₀ aralkyl such as benzyl, benzhydryl and trityl.

The substituent of the "optionally substituted aralkyl" represented by R⁴ includes the same substituents as those of the "optionally substituted aryl" as an examaple of the "optionally substituted homocyclic group" represented by Q, and the optionally substituted aralkyl may have 1 to 6, preferably 1 to 3 substituents at substitutable positions.

The cycloalkyl of the "optionally substituted cycloalkyl" represented by R⁴ includes C₃₋₆ cycloalkyl such as cyclopropyl, cyclobutyl, cyclopentyl and cyclohexyl.

The substituent of the "optionally substituted cycloalkyl" represented by R⁴ includes the same substituents as those of the "optionally substituted aryl" in the definition of the "optionally substituted homocyclic group" represented by Q, and the optionally substituted cycloalkyl may have 1 to 6, preferably 1 to 3 substituents at substitutable positions.

The "C₁₋₆ alkyl" of the "C₁₋₆ alkyl optionally substituted with (i) a group linking via a sulfur atom or (ii) a group linking via an oxygen atom" represented by R⁵ includes C₁₋₆ alkyl such as methyl, ethyl, propyl, isopropyl, butyl, isobutyl, sec-butyl, tert-butyl, pentyl, isopentyl, neopentyl, hexyl, isohexyl, 3-methylpentyl, neohexyl and 2,3-dimethylbutyl.

Examples of the "group linking via a sulfur atom" of the "C₁₋₆alkyl optionally substituted with (i) a group linking via a sulfur atom or (ii) a group linking via an oxygen atom" represented by R⁵ are the same as those of the "group linking via a sulfur atom" represented by R¹ or R².

Examples of the "group linking via an oxygen atom"of the "C₁₋₆ alkyl optionally substitued with (i) a group linking via a sulfur atom or (ii) a group linking via an oxygen atom" represented by R⁵ are the same as those of the "group linking via an oxygen atom" represented by R¹ or R².

Examples of the "optionally substituted heterocyclic group" represented by R⁵ are the same as those of the "optionally substituted heterocyclic group" represented by Q.

The "optionally esterified carboxyl" represented by R⁵ includes a group represented by -CO₂R¹⁰, wherein R¹⁰ is as defined above.

The "optionally thioesterified carboxyl" represented by R⁵ includes a group represented by -C(O)SR¹⁰, wherein R¹⁰ is as defined above.

The "optionally amidated carboxyl" represented by R⁵ includes a group represented by -CONR⁸R⁹, wherein R⁸ and R⁹ are as defined above.

The "optionally substituted hydrocarbon group" represented by R⁷ includes (1) optionally substituted alkyl, (2) optionally substituted cycloalkyl, (3) optionally substituted alkenyl, (4) optionally substituted aryl, and (5) optionally substituted aralkyl.

Examples of the "optionally substituted alkyl", the "optionally substituted cycloalkyl", the "optionally substituted alkenyl", the "optionally substituted aryl" and the "optionally substituted aralkyl" as the "optionally substituted hydrocarbon group" represented by R⁷ are the same as those of the "optionally substituted alkyl", the "optionally substituted cycloalkyl", the "optionally substituted alkenyl", the "optionally substituted aryl" and the "optionally substituted aralkyl" as the "optionally substituted hydrocarbon group" represented by R¹, R² or R⁶, resprectively.

R¹ is preferably optionally substituted C₆₋₁₄ aryl.

R² is preferably (1) C₁₋₆ alkyl (particularly C₁₋₃alkyl) substituted with a group linking via a nitrogen atom or (2) a group linking via a nitrogen atom.

R³ is preferably a group represented by -(CH₂)ₚQ, wherein p represents an integer of 0 to 3 and Q represents an optionally substituted homocyclic group or an optionally substituted heterocyclic group.

R⁴ is preferably (1) C₁₋₆ alkyl optionally substituted with C₁₋₆ alkoxy or (2) optionally substituted C₆₋₁₄ aryl.

R⁵ is preferably -C(O)R⁷, wherein R⁷ represents an optionally substituted hydrocarbon group.

R⁶ is preferably a hydrogen atom.

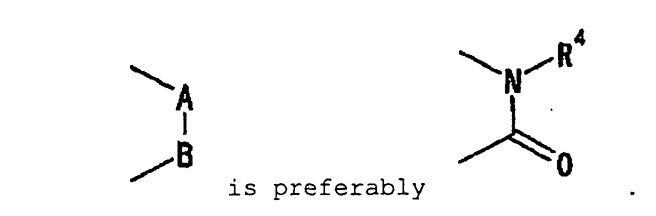

Compound (I) is preferably a compound represented by the formula: 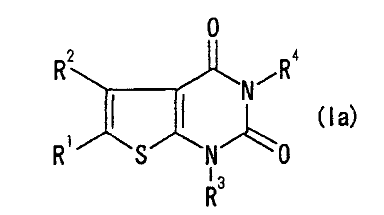 wherein respective symbols are as defined above (hereinafter, abbreviated as Compound (Ia)). Inter alia, preferred is Compound (Ia) wherein R¹ is optionally substituted C₆₋₁₄ aryl, R² is (1) C₁₋₃ alkyl substituted with a group linking via a nitrogen atom or (2) a group linking via a nitrogen atom, R³ is a group represented by -(CH₂)ₚQ (wherein p represents an integer of 0 to 3, and Q represents an optionally substituted homocyclic group or an optionally substituted heterocyclic group), and R⁴ is (1) C₁₋₆ alkyl optionally substituted with C₁₋₆ alkoxy or (2) optionally substituted C₆₋₁₄ aryl.

Inter alia, preferred is Compound (I) represented by the formula: 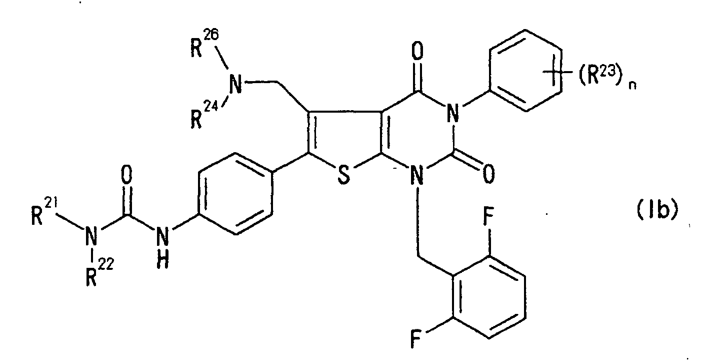 wherein R²¹ and R²² each represent (1) a hydrogen atom, (2) hydroxy, (3) C₁₋₄ alkoxy, (4) C₁₋₄ alkoxy-carbonyl or (5) optionally substituted C₁₋₄ alkyl,
R²³ represents (1) a hydrogen atom, (2) halogen, (3) hydroxy or (4) optionally substituted C₁₋₄ alkoxy, or two R²³ adjacent to each other may be taken together to form C₁₋₄ alkylenedioxy,
R²⁴ represents (1) a hydrogen atom or (2) C₁₋₄ alkyl
R²⁶ represents (1) optionally substituted C₁₋₄ alkyl or (2) a group represented by the formula: 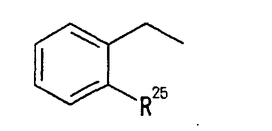 (wherein R²⁵ represents a hydrogen atom, or may be linked with R²⁴ to form a heterocycle), and
n represents an integer of 0 to 5 (hereinafter, abbreviated as Compound (Ib)).

The "C₁₋₄ alkoxy" represented by R²¹ or R²² includes methoxy, ethoxy, propoxy, isopropoxy, butoxy, isobutoxy, sec-butoxy and tert-butoxy. Among them, C₁₋₃ alkoxy is preferable, and methoxy is further preferable.

The "C₁₋₄ alkoxy-carbonyl" represented by R²¹ or R²² includes methoxycarbonyl, ethoxycarbonyl, propoxycarbonyl, isopropoxycarbonyl, butoxycarbonyl, isobutoxycarbonyl, sec-butoxycarbonyl and tert-butoxycarbonyl. Among them, C₁₋₃ alkoxy-carbonyl is preferable, and methoxycarbonyl is further preferable.

The "C₁₋₄ alkyl" of the "optionally substituted C₁₋₄ alkyl" represented by R²¹ or R²² includes straight C₁₋₄ alkyl (e.g. methyl, ethyl, propyl, butyl etc.) and branched C₃₋₄ alkyl (e.g. isopropyl, isobutyl, sec-butyl, tert-butyl etc.). Among them, C₁₋₃ alkyl is preferable and, inter alia, ethyl is preferable.

The "substituent" of the "optionally substituted C₁₋₄ alkyl" represented by R²¹ or R²² includes (i) hydroxy, (ii) C₁₋₇ acyloxy (e.g. C₁₋₆ alkyl-carbonyloxy such as acetoxy and propionyloxy), (iii) benzoyloxy, (iv) amino optionally substituted with 1 or 2 substituents selected from C₁₋₆ alkoxy-carbonyl (e.g. methoxycarbonyl, ethoxycarbonyl, tert-butoxycarbonyl etc.), benzyloxycarbonyl, C₁₋₄ acyl (e.g. C₁₋₃ alkyl-carbonyl such as acetyl and propionyl) , C₁₋₄ alkyl (e.g. methyl, ethyl, propyl, butyl etc.) and C₁₋₃ alkylsulfonyl (e.g. methansulfonyl etc.)(e.g. amino, dimethylamino, methoxycarbonylamino, ethoxycarbonylamino, tert-butoxycarbonylamino, benzyloxycarbonylamino, acetylamino, methanesufulonylamino etc.), (v) C₁₋₁₀ alkoxy (e.g. methoxy, ethoxy, propoxy, tert-butoxy etc.), (vi) C₃₋₇ cycloalkyloxycarbonyloxy-C₁₋₃ alkoxy (e.g. cyclohexyloxycarbonyloxy-1-ethoxy etc.) and (vii) C₁₋₃ alkoxy-C₁₋₃ alkoxy (e.g. methoxymethoxy, methoxyethoxy etc.). Among them, hydroxyl is preferable.

The "C₁₋₄ alkyl" of the "optionally substituted C₁₋₄ alkyl" represented by R²¹ or R²² may have 1 to 5, preferably 1 to 3 of the aforementioned substituents at substitutable positions. When the number of substituents is 2 or more, respective substituents may be the same or different.

One of R²¹ and R²² is preferably a hydrogen atom and the other is preferably C₁₋₃ alkoxy.

The "halogen" represented by R²³ includes fluorine, chlorine, bromine and iodine. Among them, chlorine is preferable.

The "C₁₋₄ alkoxy" of the "optionally substituted C₁₋₄ alkoxy" represented by R²³ includes methoxy, ethoxy, propoxy, isopropoxy, butoxy, isobutoxy, sec-butoxy and tert-butoxy. Among them, methoxy is preferable.

The "substituent" of the "optionally substituted C₁₋₄ alkoxy" represented by R²³ includes the same groups as those of the "optionally substituted C₁₋₄ alkyl" represented by R²¹ or R²². Among them, C₁₋₄alkoxy is preferable.

The C₁₋₄ alkoxy may have 1 to 5, preferably 1 to 3 of the aforementioned substituents at substitutable positions. When the number of substituents is 2 or more, respective substituents may be the same or different.

The "C₁₋₄ alkylenedioxy" formed by linking two R²³ adjacent to each other includes methylenedioxy and ethylenedioxy.

R²³ is preferably a hydrogen atom.

The "C₁₋₄ alkyl" represented by R²⁴ includes straight C₁₋₄ alkyl (e.g. methyl, ethyl, propyl, butyl, etc.) and branched C₃₋₄ alkyl (e.g. isopropyl, isobutyl, sec-butyl, tert-butyl etc.). Among them, C₁₋₃ alkyl is preferable. Inter alia, methyl is preferable.

Examples of the "optionally substituted C₁₋₄ alkyl" represented by R²⁶ are the same as those of the "optionally substituted C₁₋₄ alkyl" represented by R²¹ or R²².

The "heterocycle" formed by linking R²⁴ and R²⁵ includes a 5- or 6-membered nitrogen-containing heterocyclic group. When R²⁴ and R²⁵ are linked, a group represented by the formula: 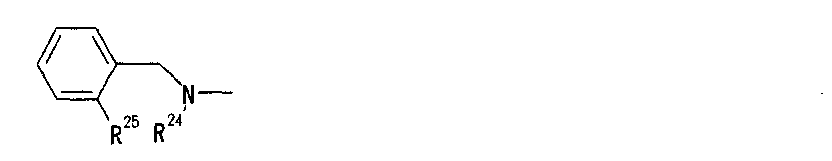 includes a group represented by the formula:

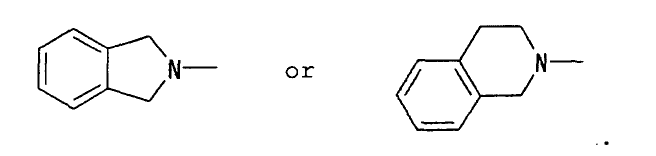

Among them, a group represented by the formula: 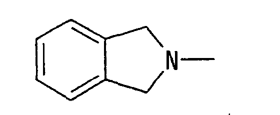 is preferable.

R²⁶ is preferably a group represented by the formula: 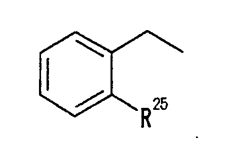 wherein R²⁵ is as defined above.

R²⁴ is preferably C₁₋₃ alkyl, and R²⁵ is preferably a hydrogen atom.

Preferably, n is an integer of 0 to 2.

Preferred Compound (I) includes a compound represented by the formula: 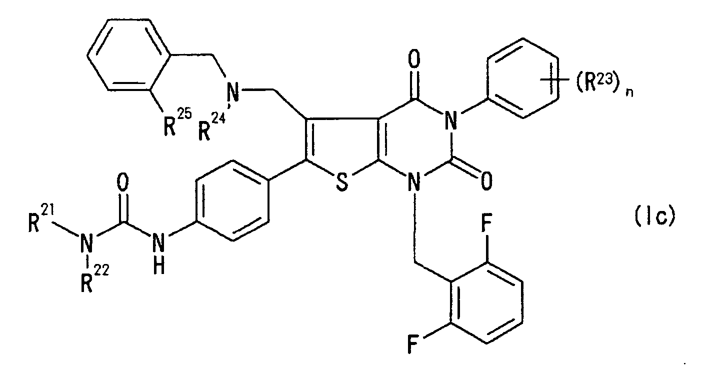 wherein respective symbols are as defined above (hereinafter, abbreviated as Compound (Ic)).

More preferred is Compound (Ic) wherein R²¹ is hydroxy, methoxy or C₁₋₃ alkyl; R²² is a hydrogen atom or C₁₋₃ alkyl; R²⁴ is C₁₋₃ alkyl; R²⁵ is a hydrogen atom; and n is 0.

Inter alia, preferred is Compound (Ic) wherein R²¹ is methoxy; R²² and R²⁵ each are a hydrogen atom; R²⁴ is C₁₋₃ alkyl; R²⁵ is a hydrogen atom; and n is 0.

In addition, preferred Compound (I) includes Compound (Ib) wherein R²¹ is (i) hydroxy, (ii) C₁₋₄ alkoxy or (iii) C₁₋₄ alkyl optionally substituted with hydroxy or C₁₋₄ alkyl-carbonyloxy; R²² is a hydrogen atom, C₁₋₄ alkyl or C₁₋₄ alkoxy-carbonyl; R²³ is a hydrogen atom, halogen, hydroxy or C₁₋₄ alkoxy-C₁₋₄ alkoxy, or two R²³ adjacent to each other are taken together to form C₁₋₃ alkylenedioxy; R²⁴ is a hydrogen atom or C₁₋₃ alkyl; R²⁶ is C₁₋₄ alkoxy-C₁₋₄ alkyl or a group represented by the formula: 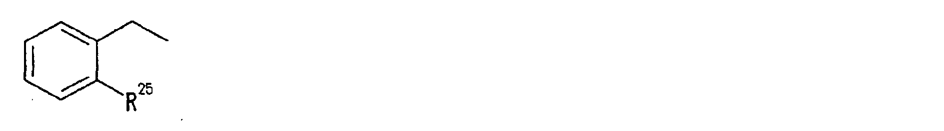 (wherein R²⁵ represents a hydrogen atom, or R²⁴ and R²⁵ are linked to form a 5- or 6-membered heterocycle); and n is 1 or 2.

Embodiment of Compound (I) includes 5-(N-benzyl-N-methylaminomethyl)-1-(2,6-difluorobenzyl)-6-[4-(3-methoxyureido)phenyl]-3-phenylthieno[2,3-d]pyrimidine-2,4(1H,3H)-dione, 5-(N-benzyl-N-methylaminomethyl)-1-(2,6-difluorobenzyl)-6-[4-(3-hydroxyureido)phenyl]-3-phenylthieno[2,3-d]pyrimidine-2,4(1H,3H)-dione, 5-(N-benzyl-N-methylaminomethyl)-1-(2,6-difluorobenzyl)-6-[4-(3-methylureido)phenyl]-3-phenylthieno[2,3-d]pyrimidine-2,4(1H,3H)-dione, 5-(N-benzyl-N-methylaminomethyl)-1-(2,6-difluorobenzyl)-6-[4-(3-ethylureido)phenyl]-3-phenylthieno[2,3-d}pyrimidine-2,4(1H,3H)-dione and their salts.

Inter alia, 5-(N-benzyl-N-methylaminomethyl)-1-(2,6-difluorobenzyl)-6-[4-(3-methoxyureido)phenyl]-3-phenylthieno[2,3-d]pyrimidine-2,4(1H,3H)-dione or a salt thereof is preferable.

A salt of Compound (I) is preferably a physiologically acceptable acid addition salt. Such a salt includes salts with inorganic acids (e.g. hydrochloric acid, hydrobromic acid, nitric acid, sulfuric acid, phosphoric acid etc.), and salts with organic acids (e.g. formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid etc.). When Compound (I) has an acidic group, it may form a physiologically acceptable salt with an inorganic base (e.g. alkali metal salt such as sodium, potassium, calcium and magnesium or alkaline earth metal, ammonia etc.) or an organic base (e.g. trimethylamine, triethylemine, pyridine, picoline, ethanolamine, diethanolamine, triethanolamine, dicyclohexylamine, N,N'-dibenzylethylenediamine etc.).

Compound (I) can be prepared by a known method such as the method described in WO95/28405, JP-A 9-169766, WO96/24597, WO97/14697, WO97/41126, WO00/00493 or WO00/56739, or the similar method.

A prodrug of Compound (I) refers to a compound which is converted into Compound (I) by a reaction with an enzyme or gastric acid in vivo.

A prodrug of Compound (I) includes, when Compound (I) has amino, a compound in which the amino is acylated, alkylated or phosphorylated (e.g. a compound obtained by eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation or tert-butylation of the amino of Compound (I)); when Compound (I) has hydroxy, a compound in which the hydroxy is acylated, alkylated, phosphorylated or borated (e.g. a compound obtained by acetylation, palmitoylation, propanoylation, pivaloylation, succinylation, fumarylation, alanylation or dimethylaminomethylcarbonylation of the hydroxy of Compond (I)); and when Compound (I) has carboxyl, a compound in which the carboxyl is esterified or amidated (e.g. a compound obtained by ethylesterification, phenylesterification, carboxymethylesterification, dimethylaminomethylesterification, pivaloyloxymethylesterification, ethoxycarbonyloxyethylesterification, phthalidylesterification, (5-methyl-2-oxo-1,3-dioxolen-4-yl)methylesterification, cyclohexyloxycarbonylethylesterification or methylamidation of the carboxyl of Compound (I)). These compounds can be prepared by a method known per se.

Alternatively, a prodrug of Compound (I) may be a compound which is converted into Compound (I) under the physiological condition as described in "Iyakuhin No Kaihatsu (Development of Drugs)", Vol. 7, Molecular Designing, published by Hirokawa Shoten, 1990, pages 163-198.

A prodrug of Compound (I) may be itself or in the form of pharmacologically acceptable salt. Such salt includes, when a prodrug of Comound (I) has an acidic group such as carboxyl, salts with inorganic bases (e.g. alkali metal such as sodium and potassium, alkaline earth metal such as calcium and magnesium, transition metal such as zinc, iron and copper) and salts with organic bases (e.g. organic amines such as trimethylamine, triethylamine, pyridine, picoline, ethanolamine, diethanolamine, triethanolamine, dicyclohexylamine and N,N'-dibenzylethylenediamine, and basic amino acids such as arginine, lysine and ornithine).

When a prodrug of Compound (I) has a basic group such as amino, the salt of the prodrug includes salts with inorganic acids or organic acids (e.g. hydrochloric acid, nitric acid, sulfuric acid, phosphoric acid, carbonic acid, bicarbonic acid, formic acid, acetic acid, propionic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid etc.), and salts with acidic amino acids such as aspartic acid and glutamic acid.

In addition, a prodrug of Compound (I) may be hydrous or anhydrous.

Compound (I) may have one or more asymmetric carbons and, regarding these asymmetric carbons, both of R configuration and S configuration are included in the present invention.

Compound (I) may be labeled with an isotope element (e.g. ³H, ¹⁴C, ³⁵S).

In addition, the non-peptidic compound having gonadotropin releasing hormone antagonistic activity includes a compound represented by the formula: 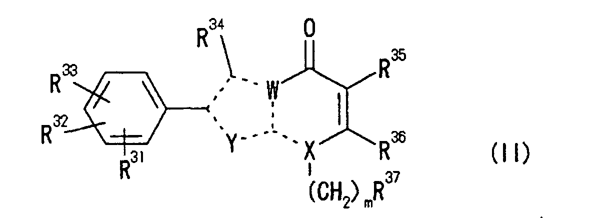 wherein one of W and Y is a nitrogen atom and the other is a carbon atom, or both of them are nitrogen atoms, X is a nitrogen atom or a carbon atom, m is an integer of 0 to 3, R³¹, R³² and R³³ are the same or different and each is (i) a hydrogen atom or (ii) a group linking via a carbon atom, a nitrogen atom, an oxygen atom or a sulfur atom, R³⁴ is a group linking via a carbon atom, R³⁵ is a hydrogen atom, halogen (e.g. fluorine, chlorine, bromine, iodine) or a group linking via a carbon atom or an oxygen atom, R³⁶ is a hydrogen atom or a group linking via a carbon atom, and R³⁷ is an optionally substituted homocyclic or an optionally substituted heterocyclic group, and the broken line represents a single bond or a double bond (hereinafter, abbreviated as Compound (II) in some cases), or a salt thereof.

Respective substituents in Compound (II) are described in detail below. In the Compound (II), the group linking via a carbon atom includes (1) an optionally substituted hydrocarbon group, (2) an optionally substituted acyl group, (3) an optionally substituted heterocyclic group linking via a carbon atom, (4) an optionally esterified or amidated carboxyl group and (5) a cyano group.

In the aforementioned formula, the group linking via a nitrogen atom includes (1) a nitro group and (2) a group represented by the formula -NR³⁸R³⁹, wherein R³⁸ represents hydrogen, an optionally substituted hydrocarbon group, an optionally substituted acyl group, optionally substituted hydroxyl, an optionally substituted heterocyclic group or a group represented by -S(O)ₜ-R⁴² (wherein t represents an integer of 0 to 2, and R⁴² represents a hydrogen atom or an optionally substituted C₁₋₁₀ hydrocarbon group), R³⁹ represents hydrogen, an optionally substituted hydrocarbon group or an optionally substituted acyl group, or R³⁸ and R³⁹ may be taken together with the adjacent atom to form an optionally substituted cyclic amino group.

In the aforementioned formula, the group linking via an oxygen atom includes optionally substituted hydroxy. The optionally substituted hydroxy is represented by the formula - OR⁴³ wherein R⁴³ represents a hydrogen atom, or an optionally substituted C₁₋₁₀ hydrocarbon, C₁₋₂₀ acyl, C₁₋₂₀ alkylsulfonyl (e.g. methylsulfonyl, ethylsulfonyl, propylsulfonyl, isopropylsulfonyl, butylsulfonyl, isobutylsulfonyl, sec-butylsulfonyl, tert-butylsulfonyl, pentylsulfonyl, hexylsulfonyl, heptylsulfonyl, octylsulfonyl, nonylsulfonyl, decylsulfonyl, undecylsulfonyl, dodecylsulfonyl, tridecylsulfonyl, tetradecylsulfonyl, pentadecylsulfonyl etc.), C₆₋₁₄ arylsulfonyl (e.g. phenylsulfonyl, 1-naphthylsulfonyl, 2-naphthylsulfonyl etc.) or heterocyclic group.

In the aforementioned formula, the group linking via a sulfur atom includes a group represented by the formula -S(O)ₜR⁴⁴ wherein t represents an integer of 0 to 2, and R⁴⁴ represents a hydrogen atom, or an optionally substituted hydrocarbon or heterocyclic group.

The optionally esterified carboxyl group includes a group represented by a formula -COOR⁵¹ wherein R⁵¹ represents a hydrogen atom or an optionally substituted C₁₋₁₀ hydrocarbon group.

The optionally amidated carboxyl group includes a group represented by the formula -CONR⁴⁵R⁴⁶ wherein R⁴⁵ represents a hydrogen atom, an optionally substituted hydrocarbon group or an alkoxy group (e.g. C₁₋₆ alkoxy such as methoxy, ethoxy, propoxy, isopropoxy, butoxy, isobutoxy, sec-butoxy, tert-butoxy, pentyloxy and hexyloxy) and R⁴⁶ represents a hydrogen atom or an optionally substituted hydrocarbon group, or R⁴⁵ and R⁴⁶ may be taken together with the adjacent nitrogen atom to form an optionally substituted cyclic amino group. The optionally amidated carboxyl group includes a group represented by -CONH₂, and a mono- or di-C₁₋₁₅ alkylcarbamoyl group, preferably a mono- or di-C₁₋₁₀ alkylcarbamoyl group (e.g. methylcarbamoyl, ethylcarbamoyl, propylcarbamoyl, isopropylcarbamoyl, butylcarbamoyl, isobutylcarbamoyl, sec-butylcarbamoyl, tert-butylcarbamoyl, pentylcarbamoyl, hexylcarbamoyl, dimethylcarbamoyl, methylethylcarbamoyl etc.).

The hydrocarbon group of the aforementioned optionally substituted hydrocarbon group is preferably a C₁₋₂₀ hydrocarbon group (preferably C₁₋₁₀ hydrocarbon group). The C₁₋₂₀ hydrocarbon group includes (1) C₁₋₁₅ alkyl (e.g methyl, ethyl, propyl, isopropyl, butyl, isobutyl, sec-butyl, tert-butyl, pentyl, hexyl, heptyl, octyl, nonyl, decyl, undecyl, dodecyl, tridecyl, tetradecyl, pentadecyl, etc.; inter alia, preferably C₁₋₁₀ alkyl, more preferably C₁₋₆ alkyl) , (2) C₃₋₁₀ cycloalkyl (e.g. cyclopropyl, cyclobutyl, cyclopentyl, cyclohexl, cycloheptyl, cyclooctyl, cyclononyl, etc.; inter alia, preferably a C₃₋₆ cycloalkyl), (3) C₂₋₁₀ alkenyl (e.g. vinyl, allyl, isopropenyl, 1-butenyl, 2-butenyl, 3-butenyl, butadienyl, 2-methylallyl, hexatrienyl, 3-octenyl, etc.; inter alia, preferably C₂₋₆ alkenyl) , (4) C₂₋₁₀ alkynyl (e.g. ethynyl, 2-propynyl, butynyl, 3-hexynyl, etc.; inter alia, preferably C₂₋₆ alkynyl) , (5) C₃₋₁₀ cycloalkenyl (e.g. cyclopropenyl, cyclopentenyl, cyclohexenyl, etc.; inter alia, preferably C₃₋₆ cycloalkenyl) , (6) C₆₋₁₄ aryl (e.g. phenyl, naphthyl, anthryl, phenanthryl, acenaphthyl, etc.; inter alia, preferably phenyl or naphthyl), and (7) C₇₋₂₀ aralkyl (e.g. C₆₋₁₄ aryl-C₁₋₆ alkyl such as benzyl, phenethyl and benzhydryl; inter alia, preferably phenyl-C₁₋₆ alkyl such as benzyl and phenethyl).

The hydrocarbon group may have 1 to 6, preferably 1 to 5, further preferably 1 to 3 substituents at substitutable positions. The substituent includes (1) halogen (e.g. fluorine, chlorine, bromine, iodine), (2) nitro, (3) nitroso, (4) cyano, (5) hydroxy optionally substituted with, for example, (i) C₁₋₆ alkyl [the C₁₋₆ alkyl may be substituted with 1 to 3 substituents selected from hydroxyl, C₁₋₆ alkoxy, C₁₋₃ alkoxy-C₁₋₃ alkoxy, C₁₋₃ alkylthio, hydroxyl-C₁₋₃alkoxy, C₁₋₆ alkyl-carbonyl, carboxy, carbamoyl, C₁₋₆ alkyl-carbamoyl, a 5- to 8-membered heterocyclic group (same as "5- or 8-membered heterocyclic group containing 1 to 4 heteroatoms selected from an oxygen atom, a sulfur atom and a nitrogen atom in addition to carbon atoms" described below) and halogen (e.g. fluorine, chlorine, bromine, iodine)], (ii) C₁₋₄acyl (e.g. C₁₋₄alkanoyl (formyl, acetyl, propionyl, butyryl, isobutyryl etc.), C₃₋₄alkenoyl (vinylcarbonyl, 1-propenylcarbonyl, 2-propenylcarbonyl etc.), (iii) C₇₋₂₀aralkyl (the C₇₋₂₀aralkyl is C₆₋₁₄ aryl-C₁₋₆ alkyl and may be substituted with 1 to 3, preferably 1 halogen (e.g. fluorine, chlorine, bromine, iodine), C₁₋₃ alkoxy or C₁₋₄alkyl, (iv) C₆₋₁₄aryl (the C₆₋₁₄aryl may be substituted with 1 to 3, preferably 1 halogen (e.g. fluorine, chlorine, bromine, iodine), (v) C₂₋₆alkenyl, (vi) C₃₋₇cycloalkyl, (vii) C₁₋₃alkoxy-carbonyl, (viii) mono- or di-C₁₋₆alkylamino, (ix) C₂₋₆alkenylamino, (x) C₁₋₆alkyl-carbonyl or (xi) C₃₋₆cycloalkyloxy-carbonyl, (6) a group represented by the formula - S(O)ₜR⁴⁷, wherein t represents an integer of 0 to 2, and R⁴⁷ represents a hydrogen atom or a hydrocarbon group optionally substituted with 1 to 3, preferably 1 substituent [e.g. halogen (e.g. fluorine, chlorine, bromine; iodine), nitro, cyano, hydroxy, oxo, thioxo, carboxy, cyano-C₆₋₁₄aryl, halogenoC₆₋₁₄aryl etc.] at a substitutable position, wherein the hydrocarbon group includes a C₁₋₂₀ hydrocarbon group, preferably, C₁₋₆alkyl, C₆₋₁₄aryl or C₇₋₂₀aralkyl, (7) an optionally substituted amino group [e.g. a group represented by the formula -NR⁴⁸R⁴⁹ wherein R⁴⁸ and R⁴⁹ are the same or different and represent C₁₋₆alkyl, C₁₋₆alkylamino-C₁₋₆alkyl, C₁₋₆alkoxy, C₂₋₆alkenyl, C₃₋₇cycloalkyl, phenyl, phenyl-C₁₋₆alkyl, C₁₋₆alkanoyl, C₃₋₆ alkenoyl, C₃₋₇cycloalkyl-carbonyl, phenyl-C₁₋₆alkyl-carbonyl, C₁₋₆alkoxy-carbonyl, phenyl-C₁₋₆alkoxy-carbonyl or a 5- to 8-membered heterocyclic group (same as "5- to 8-membered heterocyclic group containing 1 to 4 heteroatoms selected from an oxygen atom, a sulfur atom and a nitrogen atom in addition to carbon atoms" described below)], (8) a group represented by the formula -COR⁵⁰ wherein R⁵⁰ represents (i) a hydrogen atom, (ii) hydroxy, (iii) C₁₋₁₀alkyl, (iv) C₁₋₆ alkoxy (this alkoxy may be substituted with C₆₋₁₄ aryl optionally substituted with 1 to 3, preferably 1 substituent such as halogen or nitro at a substitutable position) (v) C₃₋₆cycloalkyl, (vi) C₆₋₁₄aryl, (vii) C₆₋₁₄ aryloxy, (viii) C₇₋₂₀aralkyl, or (ix) an optionally substituted amino group represented by the formula -NR⁴⁰R⁴¹ wherein R⁴⁰ represents hydrogen, an optionally substituted C₁₋₁₀hydrocarbon, C₁₋₂₀acyl, hydroxy or heterocyclic group, or a group represented by the formula -S(O)ₜ-R⁴² (wherein t represents an integer of 0 to 2, and R⁴² represents a hydrogen atom, an optionally substituted C₁₋₁₀hydrocarbon group, or a heterocyclic group), R⁴¹ represents hydrogen or a C₁₋₁₀hydrocarbon group, or R⁴⁰ and R⁴¹ may be taken together with the adjacent nitrogen atom to form an optionally substituted cyclic amino group), or (x) a 5- to 8-membered heterocyclic group (same as "5- to 8-membered heterocyclic group containing 1 to 4 heteroatoms selected from an oxygen atom, a sulfur atom and a nitrogen atom in addition to carbon atoms" described below) (e.g. preferably C₁₋₆ alkanoyl, C₃₋₆alkenoyl, C₁₋₆alkoxy-carbonyl, etc.), (9) a 5- to 8-membered heterocyclic group containing 1 to 4 heteroatoms selected from a nitrogen atom, an oxygen atom and a sulfur atom, (10) sulfo, (11) C₆₋₁₄aryl, (12) C₃₋₁₀ cycloalkyl, (13) C₁₋₆alkylenedioxy (e.g. methylenedioxy, ethylenedioxy, propylenedioxy, 2,2-dimethylenedioxy etc.), (14) oxo, (15) thioxo, (16) C₂₋₄alkynyl, (17) C₂₋₁₀alkenyl (preferably C₂₋₆alkenyl), (18) C₇₋₂₀aralkyl (e.g. C₆₋₁₄ aryl-C₁₋₆alkyl), (19) amidino and (20) azido.

Respective groups used in the explanation of a "substituent" which the aforementioned "hydrocarbon group" may have are exemplified below.

The C₁₋₁₀alkyl includes methyl, ethyl, propyl, isopropyl, butyl, isobutyl, sec-butyl, tert-butyl (i.e. C₁₋₄ alkyl), pentyl, hexyl (i.e. C₁₋₆alkyl), heptyl, octyl, nonyl, and decyl.

The C₃₋₁₀cycloalkyl includes cyclopropyl, cyclobutyl, cyclopentyl, cyclohexyl (i.e. C₃₋₆cycloalkyl), cycloheptyl (i.e. C₃₋₇cycloalkyl), cyclooctyl, cyclononyl, and cyclodecyl.

The C₂₋₁₀alkenyl includes vinyl, allyl, isopropenyl, 1-butenyl, 2-butenyl, 3-butenyl, butadienyl, 2-methylallyl, hexatrienyl (i.e. C₂₋₆alkenyl), and 3-octenyl.

The C₂₋₄alkynyl includes ethynyl, 2-propynyl, and butynyl.

The C₁₋₆alkoxy includes methoxy, ethoxy, propoxy, isopropoxy (i.e. C₁₋₃alkoxy), butoxy, isobutoxy, sec-butoxy, tert-butoxy, pentyloxy, and hexyloxy.

The C₁₋₃alkoxy-C₁₋₃alkoxy includes methoxymethoxy, methoxyethoxy, methoxypropoxy, ethoxymethoxy, ethoxyethoxy, ethoxypropoxy, propoxymethoxy, propoxyethoxy, and propoxypropoxy.

The C₁₋₃alkylthio includes methylthio, ethylthio, propylthio, and isopropylthio.

The hydroxyl-C₁₋₃alkoxy includes hydroxymethoxy, 2-hydroxyethoxy, and 3-hydroxypropoxy.

The C₁₋₆alkyl-carbonyl includes acetyl, ethylcarbonyl, propylcarbonyl, butylcarbonyl, tert-butylcarbonyl, pentylcarbonyl, and hexylcarbonyl.

The C₃₋₇cycloalkyl-carbonyl includes cyclopropylcarbonyl, cyclobutylcarbonyl, cyclopentylcarbonyl, cyclohexylcarbonyl, and cycloheptylcarbonyl.

The C₁₋₆alkoxy-carbonyl includes methoxycarbonyl, ethoxycarbonyl, propoxycarbonyl, isopropoxycarbonyl (i.e. C₁₋₃alkoxy-carbonyl), butoxycarbonyl, tert-butoxycarbonyl, pentyloxycarbonyl and hexyloxycarbonyl.

The C₃₋₆cycloalkyloxy-carbonyl includes cyclopropyloxycarbonyl, cyclobutyloxycarbonyl, cyclopentyloxycarbonyl, and cyclohexyloxycarbonyl.

The phenyl-C₁₋₆alkyl-carbonyl includes benzylcarbonyl, and phenethylcarbonyl.

The phenyl-C₁₋₆alkoxy-carbonyl includes benzyloxycarbonyl, and phenethyloxycarbonyl.

The C₁₋₆alkyl-carbamoyl includes methylcarbamoyl, ethylcarbamoyl, propylcarbamoyl, isopropylcarbamoyl, butylcarbamoyl, isobutylcarbamoyl, sec-butylcarbamoyl, tert-butylcarbamoyl, pentylcarbamoyl and hexylcarbamoyl.

The C₁₋₆alkanoyl includes formyl, acetyl, propionyl, butyryl, and isobutryl.

The C₃₋₆alkenoyl includes vinylcarbonyl, 1-propenylcarbonyl, 2-propenylcarbonyl, 1-butenylcarbonyl, and 1-pentenylcarbonyl.

The C₆₋₁₄aryl includes sphenyl, naphthyl, anthryl, phenanthryl, and acenaphthyl.

The cyanoC₆₋₁₄aryl includes 2-cyanophenyl, 3-cyanophenyl, and 4-cyanophenyl.

The halogenoC₆₋₁₄aryl includes 2-fluorophenyl, 3-fluorophenyl, 4-fluorophenyl, 2-chlorophenyl, 3-chlorophenyl, 4-chlorophenyl, 2-bromophenyl, 3-bromophenyl, 4-bromophenyl, 2,6-difluorophenyl, 2,3-dichlorophenyl, 2,4-dichlorophenyl, 2,5-dichlorophenyl, and 2,6-dichlorophenyl.

The C₇₋₂₀aralkyl, that is, C₆₋₁₄aryl-C₁₋₆alkyl includes benzyl and phenethyl.

The C₆₋₁₄aryloxy includes phenoxy, 1-naphthyloxy, and 2-naphthyloxy.

The mono- or di-C₁₋₆alkylamino includes methylamino, ethylamino, propylamino, isopropylamino, butylamino, dimethylamino, and diethylamino.

The C₂₋₆alkenylamino includes vinylamino, allylamino, isopropenylamino, 1-butenylamino, 2-butenylamino, 3-butenylamino, butadienylamino, and 2-methylallylamino.

The C₁₋₆alkylamino-C₁₋₆alkyl includes methylaminomethyl, ethylaminomethyl, propylaminomethyl, methylaminoethyl, and ethylaminoethyl.

The phenyl-C₁₋₆alkyl includes benzyl, and phenethyl.

Among the aforementioned substituents on a substituted hydrocarbon group, (9) a 5- to 8-membered heterocyclic group containing 1 to 4 heteroatoms selected from a nitrogen atom, an oxygen atom and a sulfur atom, (11) C₆₋₁₄ aryl, (12) C₃₋₁₀cycloalkyl, (16) C₂₋₄alkynyl, (17) C₂₋₁₀ alkenyl and (18) C₇₋₂₀aralkyl may further have 1 to 4, preferably 1 to 3 substituents at substitutable positions. The further substituents may be, for example, 1 to 3 groups, further preferably 1 or 2 groups selected from (1) hydroxy, (2) amino, (3) mono- or di-C₁₋₄alkylamino (e.g. methylamino, ethylamino, propylamino, dimethylamino, diethylamino etc.), (4) C₁₋₄alkoxy (e.g. methoxy, ethoxy, propoxy, isopropoxy, butoxy, isobutoxy, sec-butoxy, tert-butoxy etc.), (5) halogen (e.g. fluorine, chlorine, bromine, iodine), (6) nitro and (7) C₁₋₆alkyl (e.g. methyl, ethyl, propyl, isopropyl, butyl, isobutyl, sec-butyl, tert-butyl, pentyl, hexyl etc.).

When the hydrocarbon group is C₃₋₁₀cycloalkyl, C₃₋₁₀ cycloalkenyl, C₆₋₁₄aryl or C₇₋₂₀aralkyl, it may be substituted with 1 to 3 C₁₋₆alkyl (e.g. methyl, ethyl, propyl, isopropyl, butyl, isobutyl, sec-butyl, tert-butyl, pentyl, hexyl etc.), and this C₁₋₆alkyl may be further substituted with 1 to 3 hydroxyl, oxo, C₁₋₆alkoxy (e.g. methoxy, ethoxy, propoxy, isopropoxy, butoxy, isobutoxy, sec-butoxy, tert-butoxy, pentyloxy, hexyloxy etc.), C₁₋₃ alkylthio (e.g. methylthio, ethylthio, propylthio, isopropylthio etc.), halogen (e.g. fluorine, chlorine, bromine, iodine), carbamoyl, etc..

The substituted C₁₋₆alkyl includes formyl (wherein methyl is substituted with oxo), carboxyl (wherein methyl is substituted with oxo and hydroxy), C₁₋₆alkoxycarbonyl (wherein methyl is substituted with oxo and alkoxy) (e.g. C₁₋₆alkoxycarbonyl such as methoxycarbonyl, ethoxycarbonyl, and tert-butoxycarbonyl), hydroxyC₁₋₆alkyl (e.g. hydroxymethyl, hydroxyethyl, hydroxybutyl, hydroxypropyl etc.) and C₁₋₃alkoxy-C₁₋₆alkyl (e.g. methoxymethyl, ethoxymethyl, ethoxybutyl, propoxymethyl, propoxyhexyl etc.).

The number of the aforementioned substituents is 1 to 6, preferably 1 to 5, particularly preferably 1 to 3, most preferably 1 or 2. The number of substituents that the aforementioned substituents may further have is preferably 1 to 4, particularly preferably 1 to 3, most preferably 1 or 2.

For the group linking via a carbon atom, the acyl group of the optionally substituted acyl group in the definition of R³⁸ and R³⁹ includes a C₁₋₂₀acyl group such as formyl, C₁₋₆alkyl-carbonyl (e.g. acetyl, ethylcarbonyl, propylcarbonyl, tert-butylcarbonyl etc.), C₁₋₆alkoxycarbonyl (e.g. methoxycarbonyl, ethoxycarbonyl, tert-butoxycarbonyl etc.), C₆₋₁₄aryl-carbonyl (e.g. benzoyl, naphthoyl etc.), C₆₋₁₄aryloxy-carbonyl (e.g. phenoxycarbonyl etc.), C₇₋₁₅aralkyl-carbonyl (e.g. C₆₋₁₄aryl-C₁₋₆alkyl-carbonyl such as benzylcarbonyl etc.), C₇₋₁₉ aralkyloxycarbonyl (e.g. C₆₋₁₄aryl-C₁₋₆alkoxy-carbonyl such as benzyloxycarbonyl etc.), C₂₋₄alkenyl-carbonyl (e.g. 2-propenylcarbonyl etc.), C₃₋₆cycloalkyl-carbonyl (e.g. cyclopropylcarbonyl etc.), tricyclic C₉₋₁₀bridging cyclic hydrocarbon-carbonyl (e.g. adamantylcarbonyl etc.), heterocycle-carbonyl (e.g. (1) 5-membered heterocycle-carbonyl containing 1 to 4 heteroatoms selected from an oxygen atom, a sulfur atom and a nitrogen atom in addition to carbon atoms, such as thienylcarbonyl, furylcarbonyl, pyrrolylcarbonyl, pyrrolinylcarbonyl, oxazolylcarbonyl, thiazolylcarbonyl, pyrazolylcarbonyl, imidazolylcarbonyl, imidazolinylcarbonyl, isoxazolylcarbonyl, isothiazolylcarbonyl, 1,2,4-oxadiazolylcarbonyl, 1,3,4-oxadiazolylcarbonyl, furazanylcarbonyl, 1,2,4-thiadiazolylcarbonyl, 1,2,3-thiadiazolylcarbonyl, 1,2,5-thiadiazolylcarbonyl, 1,2,3-triazolylcarbonyl, 1,2,4-triazolylcarbonyl, triazinylcarbonyl, triazolizinylcarbonyl, and 1H- or 2H-tetrazolylcarbonyl; (2) 6-membered heterocycle-carbonyl containing 1 to 4 heteroatoms selected from an oxygen atom, a sulfur atom and a nitrogen atom in addition to carbon atoms, such as pyridylcarbonyl, pyrimidinylcarbonyl, thiomorpholinylcarbonyl, morpholinylcarbonyl, triazinylcarbonyl, pyrrolidinylcarbonyl, piperidinylcarbonyl, pyranylcarbonyl, thiopyranylcarbonyl, 1,4-oxazinylcarbonyl, 1,4-thiazinylcarbonyl, 1,3-thiazinylcarbonyl, piperazinylcarbonyl, triazinylcarbonyl, oxotriazinylcarbonyl, pyridazinylcarbonyl and pyrazinylcarbonyl, etc.), carbamoyl, N-C₁₋₆alkyl-carbamoyl (e.g. methylcarbamoyl, ethylcarbamoyl, propylcarbamoyl, isopropylcarbamoyl, butylcarbamoyl, isobutylcarbamoyl, tert-butylcarbamoyl, pentylcarbamoyl, hexylcarbamoyl etc.), and N,N-di-C₁₋₆alkyl-carbamoyl (e.g. dimethylcarbamoyl, diethylcarbamoyl, dipropylcarbamoyl, diisopropylcarbamoyl, dibutylcarbamoyl etc.).

The substituent of the optionally substituted acyl group includes the same substituents as those of the aforementioned optionally substituted hydrocarbon group.

In the Compound (II), the heterocyclic group of the heterocyclic group or the optionally substituted heterocyclic group includes a 5- to 8-membered heterocyclic group containing 1 to 4 heteroatoms selected from an oxygen atom, a sulfur atom and a nitrogen atom in addition to carbon atoms, a dicyclic or tricyclic fused heterocyclic group formed by fusing 2 or 3 of said heterocyclic groups which may be the same or different, and a dicyclic or tricyclic fused heterocycle group formed by fusing said heterocyclic group with 1 or 2 benzene rings.

Embodiment of the heterocyclic group includes (1) a 5-membered heterocyclic group containing 1 to 4 heteroatoms selected from an oxygen atom, a sulfur atom and a nitrogen atom in addition to carbon atoms, such as thienyl, furyl, pyrrolyl, pyrrolinyl, oxazolyl, thiazolyl, pyrazolyl, imidazolyl, imidazolinyl, isoxazolyl, isothiazolyl, 1,2,4-oxadiazolyl, 1,3,4-oxadiazolyl, furazanyl, 1,2,4-thiadiazolyl, 1,2,3-thiadiazolyl, 1,2,5-thiadiazolyl, 1,2,3-triazolyl, 1,2,4-triazolyl, triazinyl, triazolizinyl, and 1H- and 2H-tetrazolyl; (2) a 6-membered heterocyclic group containing 1 to 4 heteroatoms selected from an oxygen atom, a sulfur atom and a nitrogen atom in addition to carbon atoms such as pyridyl, pyrimidinyl, thiomorpholinyl, morpholinyl, triazinyl, pyrrolidinyl, piperidinyl, pyranyl, thiopyranyl, 1,4-oxazinyl, 1,4-thiazinyl, 1,3-thiazinyl, piperazinyl, triazinyl, oxotriazinyl, pyridazinyl and pyrazinyl; and (3) a dicyclic or tricyclic fused heterocyclic group containing 1 to 4 heteroatoms selected from an oxygen atom, a sulfur atom and a nitrogen atom in addition to carbon atoms, such as benzofuryl, benzothiazolyl, benzoxazolyl, tetrazolo[1,5-b]pyridazinyl, triazolo[4,5-b]pyridazinyl, benzimidazolyl, quinolyl, isoquinolyl, cinnolinyl, phthalazinyl, quinazolinyl, quinoxalinyl, indolizinyl, indolyl, quinolizinyl, 1,8-naphthyridinyl, purinyl, pteridinyl, dibenzofuranyl, carbazolyl, acridinyl, phenanthridinyl, chromanyl, benzoxazinyl, phenazinyl, phenothiazinyl and phenoxazinyl.

The substituent which the heterocyclic group may have includes (1) C₁₋₆alkyl (e.g. methyl, ethyl, propyl, isopropyl, butyl, isobutyl, sec-butyl, tert-butyl, pentyl, hexyl etc.), (2) C₂₋₆alkenyl (e.g. vinyl, allyl, isopropenyl, 1-butenyl, 2-butenyl, 3-butenyl, butadienyl, 2-methylallyl, hexatrienyl etc.), (3) C₂₋₆alkynyl (e.g. ethynyl, 2-propynyl, butynyl, 3-hexynyl etc.), (4) C₃₋₆ cycloalkyl (e.g. cyclopropyl, cyclobutyl, cyclopentyl, cyclohexyl etc.), (5) C₅₋₇cycloalkenyl (e.g. cyclopentenyl, cyclohexenyl, cycloheptenyl etc.), (6) C₇₋₁₁aralkyl (e.g. C₆₋₁₀ aryl-C₁₋₅ alkyl such as benzyl and phenethyl, preferably benzyl), (7) C₆₋₁₄aryl (e.g. phenyl, naphthyl, anthryl, phenanthryl, acenaphthyl, anthracenyl etc., preferably phenyl), (8) C₁₋₆alkoxy (e.g. methoxy, ethoxy, propoxy, isopropoxy, butoxy, isobutoxy, sec-butoxy, tert-butoxy, pentyloxy, hexyloxy etc.), (9) C₆₋₁₄aryloxy (e.g. phenoxy etc.), (10) C₁₋₆alkanoyl (e.g. formyl, acetyl, propionyl, butyryl, isobutyryl etc.), (11) C₆₋₁₄aryl-carbonyl (e.g. benzoyl etc.), (12) C₁₋₆alkanoyloxy (e.g. formyloxy, acetyloxy, propionyloxy, butyryloxy, isobutyryloxy etc.), (13) C₆₋₁₄aryl-carbonyloxy (e.g. benzoyloxy etc.), (14) carboxyl, (15) C₁₋₆alkoxy-carbonyl (e.g. methoxycarbonyl, ethoxycarbonyl, propoxycarbonyl, isopropoxycarbonyl, butoxycarbonyl, isobutoxycarbonyl, tert-butoxycarbonyl etc.), (16) carbamoyl, (17) N-mono-C₁₋₄alkylcarbamoyl (e.g. N-methylcarbamoyl, N-ethylcarbamoyl, N-propylcarbamoyl, N-isopropylcarbamoyl, N-butylcarbamoyl etc.), (18) N,N-di-C₁₋₄ alkylcarbamoyl (e.g. N,N-dimethylcarbamoyl, N,N-diethylcarbamoyl, N,N-dipropylcarbamoyl, N,N-dibutylcarbamoyl etc.), (19) 3- to 6-membered cyclic aminocarbonyl (e.g. 1-aziridinylcarbonyl, 1-azetidinylcarbonyl, 1-pyrrolidinylcarbonyl, 1-piperidinylcarbonyl, N-methylpiperazinylcarbonyl, morpholinocarbonyl etc.), (20) halogen (e.g. fluorine, chlorine, bromine, iodine), (21) mono-, di- or tri-halogeno-C₁₋₄alkyl (e.g. chloromethyl, dichloromethyl, trifluoromethyl, trifluoroethyl etc.), (22) oxo, (23) amidino, (24) imino, (25) amino, (26) mono- or di-C₁₋₄ alkylamino (e.g. methylamino, ethylamino, propylamino, isopropylamino, butylamino, dimethylamino, diethylamino, dipropylamino, diisopropylamino, dibutylamino etc.), (27) a 3- to 6-membered cyclic amino group optionally containing 1 to 3 heteroatoms selected from an oxygen atom, a sulfur atom and a nitrogen atom in addition to carbon atoms and one nitrogen atom (e.g. aziridinyl, azetidinyl, pyrrolidinyl, pyrrolinyl, pyrrolyl, imidazolyl, pyrazolyl, imidazolidinyl, piperidino, morpholino, dihydropyridyl; N-methylpiperazinyl, N-ethylpiperazinyl etc.), (28) C₁₋₆ alkanoylamino (e.g. formamido, acetamido, trifluoroacetamido, propionylamido, butyrylamido, isobutyrylamido etc.), (29) benzamido, (30) carbamoylamino, (31) N-C₁₋₄alkylcarbamoylamino (e.g. N-methylcarbamoylamino, N-ethylcarbamoylamino, N-propylcarbamoylamino, N-isopropylcarbamoylamino, N-butylcarbamoylamino etc.), (32) N,N-di-C₁₋₄alkylcarbamoylamino (e.g. N,N-dimethylcarbamoylamino, N,N-diethylcarbamoylamino, N,N-dipropylcarbamoylamino, N,N-dibutylcarbamoylamino etc.), (33) C₁₋₃alkylenedioxy (e.g. methylenedioxy, ethylenedioxy etc.), (34) -B(OH)₂, (35) hydroxy, (36) epoxy (-O-) , (37) nitro, (38) cyano, (39) mercapto, (40) sulfo, (41) sulfino, (42) phosphono, (43) sulfamoyl, (44) C₁₋₆alkylsulfamoyl (e.g. N-methylsulfamoyl, N-ethylsulfamoyl, N-propylsulfamoyl, N-isopropylsulfamoyl, N-butylsulfamoyl etc.), (45) diC₁₋₆ alkylsulfamoyl (e.g. N,N-dimethylsulfamoyl, N,N-diethylsulfamoyl, N,N-dipropylsulfamoyl, N,N-dibutylsulfamoyl etc.), (46) C₁₋₆alkylthio (e.g. methylthio, ethylthio, propylthio, isopropylthio, n-butylthio, sec-butylthio, tert-butylthio etc.), (47) phenylthio, (48) C₁₋₆ alkylsulfinyl (e.g. methylsulfinyl, ethylsulfinyl, propylsulfinyl, butylsulfinyl etc.), (49) phenylsulfinyl, (50) C₁₋₆alkylsulfonyl (e.g. methylsulfonyl, ethylsulfonyl, propylsulfonyl, butylsulfonyl etc.) and (51) phenylsulfonyl.

The number of substituents which the heterocyclic group may have is 1 to 6, preferably 1 to 3, further preferably 1 or 2.

The heterocyclic group of the optionally substituted heterocyclic group linking via a carbon atom includes a 5- to 8-membered heterocyclic group containing 1 to 4 heteroatoms selected from an oxygen atom, a sulfur atom and a nitrogen atom in addition to carbon atoms, a dicyclic or tricyclic fused heterocyclic group formed by fusing 2 or 3 of said heterocyclic groups which may be the same or different, and a dicyclic or tricyclic fused heterocyclic group formed by fusing said heterocyclic group with 1 or 2 benzene rings, wherein said heterocyclic group is linked via one of carbon atoms constituting the heterocycle.

Embodiment of the heterocyclic group linking via a carbon atom includes (1) a 5-membered heterocyclic group containing 1 to 4 heteroatoms selected from an oxygen atom, a sulfur atom and a nitrogen atom in addition to carbon atoms, such as thienyl (e.g. 2- or 3-thienyl), furyl (e.g. 2- or 3-furyl), pyrrolyl (e.g. 2- or 3-pyrrolyl), oxazolyl (e.g. 2-, 4- or 5-oxazolyl), thiazolyl (e.g. 2-, 4- or 5-thiazolyl), pyrazolyl (e.g. 3-, 4- or 5-pyrazolyl), pyrrolidinyl (e.g. 2- or 3-pyrrolidinyl), imidazolyl (e.g. 2-, 4- or 5-imidazolyl), imidazolinyl (e.g. 2-imidazolinyl, 4-imidazolidinyl), isoxazolyl (e.g. 3-, 4- or 5-isoxazolyl), isothiazolyl (e.g. 3-, 4- or 5-isothiazolyl), oxadiazolyl [e.g. 3- or 5-(1,2,4-oxadiazolyl), 2-, 5- or 6-(1,3,4-oxadiazolyl)], thiadiazolyl [e.g. 3- or 5-(1,2,4-thiadiazolyl), 2- or 5-(1,3,4-thiadiazolyl), 4- or 5-(1,2,3-thiadiazolyl), 3- or 4-(1,2,5-thiadiazolyl)], triazolyl [e.g. 2- or 5-(1,2,3-triazolyl), 3- or 5-(1,2,4-triazolyl)], and tetrazolyl [e.g. 5-(1H- or 2H-tetrazolyl)]; (2) a 6-membered heterocyclic group containing 1 to 4 heteroatoms selected from an oxygen atom, a sulfur atom and a nitrogen atom in addition to carbon atoms, such as pyridyl (e.g. 2-, 3- or 4-pyridyl), pyrimidinyl (e.g. 2-, 4- or 5-pyrimidinyl), thiomorpholinyl (e.g. 2- or 3-thiomorpholinyl), morpholinyl (e.g. 2- or 3-morpholinyl), triazinyl (e.g. 3- or 6-triazinyl), piperidinyl (e.g. 2-, 3- or 4-piperidinyl), pyranyl (e.g. 2- or 3-pyranyl), thiopyranyl (e.g. 2- or 3-thiopyranyl), oxazinyl [e.g. 2- or 3-(1,4-oxazinyl)], thiazinyl [e.g. 2-or 3-(1,4-thiazinyl), 1- or 4-(1,3-thiazinyl)], piperazinyl (e.g. 2- or 3-piperazinyl), triazinyl (e.g. 3- or 6-triazinyl), pyridazinyl (e.g. 3- or 4-pyridazinyl), pyrazinyl (e.g. 2- or 3-pyrazinyl), and pyridazinyl (e.g. 3- or 4-pyridazinyl); and (3) a dicyclic or tricyclic fused heterocyclic group containing 1 to 4 heteroatoms selected from an oxygen atom, a sulfur atom and a nitrogen atom in addition to carbon atoms, such as benzofuryl, benzothiazolyl, benzoxazolyl, tetrazolo[1,5-b]pyridazinyl, triazolo[4,5-b]pyridazinyl, benzimidazolyl, quinolyl, isoquinolyl, cinnolinyl, phthalazinyl, quinazolinyl, quinoxalinyl, indolidinyl, indolyl, quinolizinyl, 1,8-naphthyridinyl, purinyl, pteridinyl, dibenzofuranyl, carbazolyl, acridinyl, phenanthridinyl, chromanyl, benzoxazinyl, phenazinyl, phenothiazinyl and phenoxazinyl.

The substitutent which the heterocyclic group linking via a carbon atom may have includes the same substituents as those of the aforementioned optionally substituted heterocyclic group.

The cyclic amino group of the aforementioned cyclic amino group or the aforementioned optionally substituted cyclic amino group includes a 5- to 7-membered nitrogen-containing cyclic group optionally further having one atom selected from an oxygen atom, a sulfur atom and a nitrogen atom, for example, pyrrolidinyl, pyrrolinyl, pyrrolyl, pyrazolidinyl, pyrazolinyl, pyrazolyl, imidazolidinyl, imidazolinyl, imidazolyl, 1,2,3-triazinyl, 1,2,3-triazolidinyl, 1,2,3-triazolyl, 1,2,3,4-tetrazolyl, piperidinyl, piperazinyl, azepinyl, hexamethyleneimino, oxazolidino, morpholino, thiazolidino and thiomorpholino. Inter alia, a 5- or 6-membered group is preferable. For example, pyrrolidinyl, pyrazolinyl, pyrazolyl, piperidinyl, piperazinyl, morpholino, or thiomorpholino is preferable.

The cyclic amino group may have 1 to 3 substituents at substitutable positions, and the substituent includes (1) C₁₋₆alkyl (e.g. methyl, ethyl, propyl, isopropyl, butyl, isobutyl, sec-butyl, tert-butyl, pentyl, hexyl etc.), (2) C₆₋₁₄aryl (e.g. phenyl, naphthyl, anthryl, phenanthryl, acenaphthyl etc.), (3) C₇₋₁₀aralkyl (phenyl-C₁₋₄alkyl (e.g. benzyl, phenethyl etc.)), (4) benzhydryl, (5) C₁₋₆alkyl-carbonyl (e.g. acetyl, propionyl etc.), (6) C₆₋₁₄aryl-carbonyl (e.g. benzoyl etc.) and (7) C₁₋₆alkoxy-carbonyl (e.g. methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, hexyloxycabonyl etc.). A preferable example of the substituent is C₁₋₆alkyl. Inter alia, C₁₋₃alkyl is further preferable.

The homocyclic group of the optionally substituted homocyclic group includes an optionally fused 3- to 7-membered carbocyclic group such as a C₆₋₁₀aryl group (e.g. phenyl, naphthyl etc.), a C₃₋₇cycloalkyl group (e.g. cyclopropyl, cyclobutyl, cyclopentyl, cyclohexyl, cycloheptyl etc.), and C₃-₇cycloalkenyl (e.g. cyclopropenyl, cyclobutenyl, cyclopentenyl, cyclohexenyl, cycloheptenyl etc.).

The homocyclic group may have 1 to 6, preferably 1 to 3, further preferably 1 or 2 substituents at substitutable positions. The substituent includes (1) C₁₋₁₅alkyl (e.g. methyl, ethyl, propyl, isopropyl, butyl, isobutyl, sec-butyl, tert-butyl, pentyl, hexyl, heptyl, octyl, nonyl, decyl, undecyl, dodecyl, tridecyl, tetradecyl, pentadecyl etc.) optionally substituted with 1 to 3, preferably 1 or 2 halogen (e.g. fluorine, chlorine, bromine, iodine) (preferably, C₁₋₆alkyl optionally substituted with halogen), (2) C₃₋₁₀cycloalkyl (e.g. cyclopropyl, cyclobutyl, cyclopentyl, cyclohexyl, cycloheptyl, cyclooctyl, cyclononyl, cyclodecyl etc.), (3) C₂₋₁₀alkenyl (e.g. vinyl, allyl, isopropenyl, 1-butenyl, 2-butenyl, 3-butenyl, butadienyl, 2-methylallyl, hexatrienyl, 3-octenyl etc.), (4) C₂₋₁₀alkynyl (e.g. ethynyl, 2-propynyl, butynyl, 3-hexynyl etc.), (5) C₃₋₁₀cycloalkenyl (e.g. cyclopropenyl, cyclopentenyl, cyclohexenyl etc.), (6) C₆₋₁₀aryl (e.g. phenyl, naphthyl etc.), (7) C₇₋₂₀aralkyl (e.g. benzyl, phenethyl etc.), (8) nitro, (9) hydroxyl, (10) mercapto, (11) oxo, (12) thioxo, (13) cyano, (14) carbamoyl, (15) carboxyl, (16) C₁₋₆alkoxy-carbonyl (e.g. methoxycarbonyl, ethoxycarbonyl etc.), (17) sulfo, (18) halogen (e.g. fluorine, chlorine, bromine, iodine), (19) C₁₋₆alkoxy (e.g. methoxy, ethoxy, propoxy, isopropoxy, butoxy, isobutoxy, sec-butoxy, tert-butoxy, pentyloxy, hexyloxy etc.), (20) C₆₋₁₀aryloxy (e.g. phenoxy etc.), (21) C₁₋₆acyloxy (e.g. C₁₋₆ alkanoyloxy such as acetoxy, propionyloxy etc.), (22) C₁₋₆ alkylthio (e.g. methylthio, ethylthio, propylthio, isopropylthio, butylthio, tert-butylthio etc.), (23) C₆₋₁₀ arylthio (e.g. phenylthio etc.), (24) C₁₋₆alkylsulfinyl (e.g. methylsulfinyl, ethylsulfinyl etc.), (25) C₆₋₁₀arylsulfinyl (e.g. phenylsulfinyl etc.), (26) C₁₋₆alkylsulfonyl (e.g. methylsulfonyl, ethylsulfonyl etc.), (27) C₆₋₁₀arylsulfonyl (e.g. phenylsulfonyl etc.), (28) amino, (29) C₁₋₆acylamino (e.g. C₁₋₆alkanoylamino such as acetylamino, propionylamino etc.), (30) mono- or di-C₁₋₄alkylamino (e.g. methylamino, ethylamino, propylamino, isopropylamino, butylamino, dimethylamino, diethylamino etc.), (31) C₃₋₈cycloalkylamino (e.g. cyclopropylamino, cyclobutylamino, cyclopentylamino, cyclohexylamino etc.), (32) C₆₋₁₀arylamino (e.g. anilino etc.), (33) C₁₋₆alkanoyl (e.g. formyl, acetyl, hexanoyl etc.), (34) C₆₋₁₀aryl-carbonyl (e.g. benzoyl etc.), and (35) a 5- or 6-membered heterocyclic group containing 1 to 4 heteroatoms selected from oxygen, sulfur and nitrogen in addition to carbon atoms [e.g. thienyl (e.g. 2- or 3-thienyl), furyl (e.g. 2- or 3-furyl), pyrazolyl (e.g. 3-, 4- or 5-pyrazolyl), thiazolyl (e.g. 2-, 4- or 5-thiazolyl), isothiazolyl (e.g. 3-, 4- or 5-isothiazolyl), oxazolyl (e.g. 2-, 4- or 5-oxazolyl), isoxazolyl (e.g. 3-, 4- or 5-isoxazolyl), imidazolyl (e.g. 2-, 4- or 5-imidazolyl), triazolyl (e.g. 1,2,3- or 1,2,4-triazolyl), tetrazolyl (e.g. 1H or 2H-tetrazolyl), pyridyl (e.g. 2-, 3- or 4-pyridyl), pyrimidinyl (e.g. 2-, 4- or 5-pyrimidinyl), pyridazinyl (e.g. 3- or 4-pyridazinyl), quinolyl, isoquinolyl, indolyl etc.].

The optionally substituted hydroxy represented by R³⁸ or R⁴⁰ includes the aforementioned group represented by the formula -OR⁴³ wherein R⁴³ is as defined above.

In the aforementioned formula, R³¹, R³² and R³³ are the same or different and preferably each is (i) hydrogen or (ii) the aforementioned group linking via a carbon atom, a nitrogen atom or an oxygen atom. Inter alia, preferably, R³¹ is an optionally substituted C₁₋₁₅alkyl, C₃₋₁₀cycloalkyl, C₂₋₁₀ alkenyl, C₂₋₁₀alkynyl, C₃₋₁₀cycloalkenyl, C₆₋₁₄aryl, C₇₋₂₀aralkyl or C₁₋₂₀acyl group, a nitro group, a group. represented by the formula -NR⁴⁰R⁴¹ wherein R⁴⁰ and R⁴¹ are as defined above, or a group represented by the formula -OR⁴³ wherein R⁴³ represents a hydrogen atom, or an optionally substituted C₁₋₁₀hydrocarbon, C₁₋₂₀acyl, C₁₋₂₀ alkylsulfonyl, C₆₋₁₄arylsulfonyl or a 5- to 8-membered heterocyclic group (same as the aforementioned "5- to 8-membered heterocyclic group containing 1 to 4 heteroatoms selected from an oxygen atom, a sulfur atom and a nitrogen atom in addition to carbon atoms"), and at least one of R³² and R³³ is hydrogen and the other is the aforementioned group linking via a carbon atom, a nitrogen atom or an oxygen atom (preferably, both of R³² and R³³ are hydrogen).

R³¹ is preferably a C₁₋₁₀alkyl group (preferably, C₁₋₆ alkyl group) optionally substituted with 1 to 3, preferably 1 hydroxy, a nitro group, an amino group, a group represented by the formula-NR⁴⁰R⁴¹ wherein R⁴⁰ represents hydrogen, and R⁴¹ represents C₁₋₆alkyl-carbonyl optionally substituted with 1 to 3, preferably 1 hydroxy, C₁₋₆ alkylamino-carbonyl optionally substituted with 1 to 3, preferably 1 C₁₋₆alkoxy (e.g. methoxy, ethoxy, propoxy, isopropoxy, butoxy, isobutoxy, sec-butoxy, tert-butoxy, pentyloxy, hexyloxy etc.), or C₆₋₁₄arylamino-carbonyl, or a group represented by the formula -OR⁴³ wherein R⁴³ represents hydrogen, C₁₋₁₀alkyl optionally substituted with 1 to 3, preferably 1 hydroxy, C₃₋₁₀cycloalkyl, C₁₋₆alkyl-carbonyl optionally substituted with 1 to 3, preferably 1 hydroxy, C₁₋₆alkylsulfonyl, or C₆₋₁₀arylsulfonyl.

In the aforementioned formula, R³⁴ is preferably (1) an optionally substituted C₁₋₁₀hydrocarbon group, (2) an optionally substituted C₁₋₂₀acyl group, (3) an optionally substituted heterocyclic group linking via a carbon atom, (4) an optionally esterified or amidated carboxyl group or (5) a cyano group. Inter alia, preferably, R³⁴ is an optionally substituted C₁₋₁₅alkyl, C₃₋₁₀cycloalkyl, C₂₋₁₀ alkenyl, C₂₋₁₀alkynyl, C₃₋₁₀cycloalkenyl, C₆₋₁₀aryl or C₇₋₂₀ aralkyl group. Further preferably, R³⁴ is an optionally substituted C₁₋₆alkyl group (e.g. an optionally substituted aminoalkyl group). A preferable example of R³⁴ is a group represented by the formula -(CH₂)ᵤ-NR⁴⁰R⁴¹ wherein u represents an integer of 1 to 3, R⁴⁰ represents hydrogen, an optionally substituted C₁₋₁₀hydrocarbon group, an optionally substituted C₁₋₂₀acyl group, optionally substituted hydroxy (a group represented by the aforementioned formula -OR⁴³), an optionally substituted heterocyclic group, or a group represented by the formula -S(O)ₜR⁴² (wherein t represents an integer of 0 to 2, and R⁴² represents a hydrogen atom or an optionally substituted C₁₋₁₀ hydrocarbon group), and R⁴¹ represents hydrogen or a C₁₋₁₀ hydrocarbon group, or R⁴⁰ and R⁴¹ may be taken together with the adjacent nitrogen atom to form an optionally substituted cyclic amino group. More preferably, R³⁴ is halogen, hydroxy optionally substituted with a C₁₋₂₀acyl group, or a C₁₋₃alkyl group optionally substituted with an amino group optionally substituted with C₁₋₁₀alkyl and/or C₆₋₁₄ aryl-C₁₋₁₀ alkyl. Particularly preferably R³⁴ is N-C₁₋₆ alkyl-N-benzylaminomethyl.

In the aforementioned formula, the halogen represented by R³⁵ includes fluorine, chlorine, bromine and iodine.

Preferable examples of R³⁵ are hydrogen, an optionally substituted C₁₋₁₅alkyl group, an optionally substituted C₃₋₁₀ cycloalkyl group, an optionally substituted C₂₋₁₀alkenyl group, an optionally substituted C₂₋₁₀alkynyl, an optionally substituted C₃₋₁₀cycloalkenyl group, an optionally substituted C₆₋₁₄aryl group, an optionally substituted C₇₋₂₀ aralkyl group, an optionally substituted C₁₋₂₀acyl group, an optionally esterified or amidated carboxyl group, and a group represented by the formula -OR⁴³ wherein R⁴³ represents a hydrogen atom, or an optionally substituted C₁₋₁₅alkyl, C₃₋₁₀cycloalkyl, C₂₋₁₀alkenyl, C₂₋₁₀alkynyl, C₃₋₁₀ cycloalkenyl, C₆₋₁₄aryl, C₇₋₂₀aralkyl, C₁₋₂₀acyl, C₁₋₂₀ alkylsulfonyl, C₆₋₁₄arylsulfonyl or heterocyclic group. Inter alia, R³⁵ is preferably hydrogen, a C₁₋₁₅alkyl group optionally substituted with 1 to 3, preferably 1 C₆₋₁₄aryl or C₁₋₆alkoxy, C₁₋₆alkoxy-carbonyl optionally substituted with 1 to 3, preferably 1 hydroxy, C₁₋₆alkoxy-carbonyl (e.g. methoxycarbonyl, ethoxycarbonyl, tert-butoxycarbonyl etc.), C₆₋₁₄aryl-carbonyl (e.g. benzoyl etc.), C₆₋₁₄aryloxy-carbonyl (e.g. phenoxycarbonyl etc.), C₇₋₁₅aralkyl-carbonyl (e.g. benzylcarbonyl etc.), C₇₋₁₉aralkyloxy-carbonyl (e.g. benzyloxycarbonyl etc.), N-C₁₋₁₀alkyl-N- (C₁₋₁₀alkoxy) aminocarbonyl (e.g. N-methyl-N-methoxyamino-carbonyl etc.), C₁₋₁₅ alkyloxy or C₁₋₂₀arylsulfonyl. Further preferably, R³⁵ is (1) a C₁₋₆alkoxy-carbonyl group, (2) a C₆₋₁₄aryl group optionally substituted with halogen or C₁₋₆alkoxy, or (3) a phenyl-C₁₋₃alkyl group.

In the aforementioned formula, R³⁶ is preferably hydrogen, or an optionally substituted C₁₋₁₅alkyl,- C₃₋₁₀ cycloalkyl, C₂₋₁₀alkenyl, C₂₋₁₀alkynyl, C₃₋₁₀cycloalkenyl, C₆₋₁₄ aryl or C₇₋₂₀aralkyl group. Inter alia, R³⁶ is preferably hydrogen or a C₁₋₁₀alkyl group, and further preferably hydrogen or a C₁₋₆alkyl group.

In the aforementioned formula, examples of R³⁷ include an optionally substituted homocyclic group and a heterocyclic group, preferably a C₆₋₁₄aryl group. Further preferable examples of R³⁷ include a phenyl group optionally substituted with 1 to 3, preferably 1 to 2 halogen or C₁₋₆alkoxy. Particularly preferable is a phenyl group optionally substituted with 1 or 2 halogen.

In Compound (II), m is an integer of 0 to 3, preferably m is an integerof 0 to 2, further preferably m is 0 or 1.

In the aforementioned formula, u is an integer of 1 to 3, preferably, u is 1 or 2, more preferably, u is 1.

In Compound (II), one of W and Y represents a nitrogen atom and the other represents a carbon atom, both of them represent nitrogen atoms, or X represents a nitrogen atom or a carbon atom. Therefore, Compound (II) includes compounds represented by the formulas 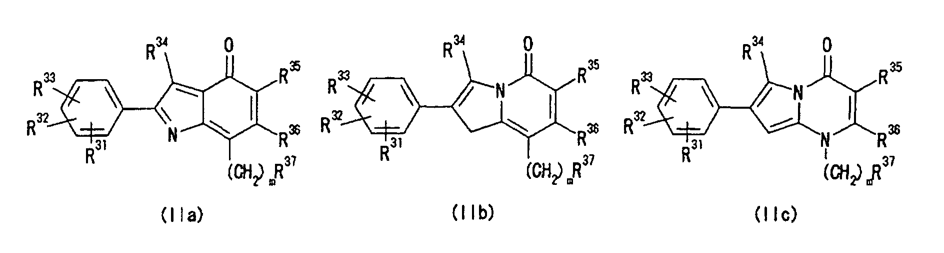 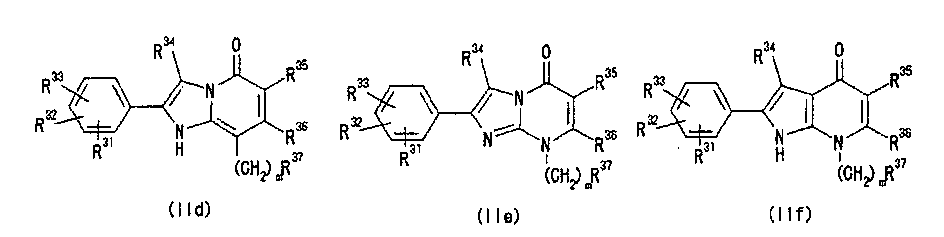 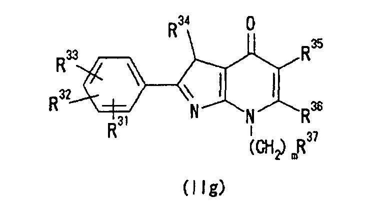 wherein respective symbols are as defined above,
(preferably compounds represented by the formulas (IIa), (IIb), (IIc), (IId), (IIe) and (IIg)). Inter alia, preferred is Compound (II) in which X is a nitrogen atom, inter alia, compounds represented by the formulas (IIc) and (IIe), most preferably, a compound represented by the formula (IIe).

Among Compound (II), preferred is a compound represented by the general formula: 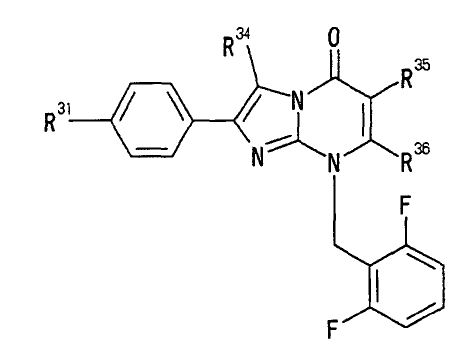 wherein respective symbols are as defined above. Inter alia, further preferred is a compound in which R³¹ is (1) an amino group optionally substituted with (i) carbamoyl optionally substituted with C₁₋₆alkyl or C₁₋₆alkoxy or (ii) C₁₋₆alkyl-carbonyl, or (2) a C₁₋₆alkoxy group optionally substituted with C₃₋₆cycloalkyl; R³⁴ is a N-C₁₋₆alkyl-N-benzylaminomethyl group; R³⁵ is (1) a C₁₋₆alkoxy-carbonyl group, (2) a C₆₋₁₄aryl group optionally substituted with halogen or C₁₋₆alkoxy, or (3) a phenyl-C₁₋₃alkyl group; and R³⁶ is a hydrogen atom.

Preferred is also a compound in which R³¹ is (1) a nitro group, (2) an amino group optionally substituted with 1 or 2 substituents selected from (i) C₁₋₆alkyl optionally substituted with hydroxy, (ii) C₁₋₆alkyl-carbonyl optionally substituted with hydroxy, halogen or thienyl, (iii) C₆₋₁₀ aryl-carbonyl optionally substituted C₁₋₆alkyl, C₁₋₆alkoxy or halogen, (iv) C₃₋₆cycloalkyl-carbonyl, (v) C₂₋₄alkenyl-carbonyl, (vi) C₁₋₆alkoxy-carbonyl, (vii) C₁₋₆alkylamino-carbonyl, (viii) C₁₋₆alkoxyamino-carbonyl, (ix) phenylaminocarbonyl, (x) isoxazolylcarbonyl, thienylcarbonyl, thiazolylcarbonyl, pyrazolylcarbonyl or furylcarbonyl, each optionally substituted with 1 or 2 substituents selected from C₁₋₆alkyl, nitro and C₁₋₆alkoxy, (xi) pyridylcarbonyl, (xii) C₁₋₆alkylsulfonyl, (xiii) thienylsulfonyl and (xiv) phenylsulfonyl optionally substituted with C₁₋₆alkyl, (3) a pyrrolyl group, or (4) a hydroxy group optionally substituted with C₁₋₆alkyl, C₃₋₆ cycloalkyl-C₁₋₃ alkyl or C₁₋₆alkyl-carbonyl; R³⁴ represents a C₁₋₆alkyl group optionally substituted with 1 or 2 substituents selected from (1) halogen, (2) hydroxy and (3) amino optionally substituted with 1 or 2 substituents selected from C₁₋₆alkyl, phenyl-C₁₋₃alkyl and di-C₁₋₆ alkylamino-C₁₋₃ alkyl; R³⁵ is (1) halogen, (2) a phenyl group optionally substituted with halogen or C₁₋₆alkyl or (3) a carbonyl group substituted with (i) C₁₋₆alkyl, (ii) amino substituted with C₁₋₆alkyl and C₁₋₆alkoxy or (iii) C₁₋₆alkoxy; and R³⁶ is a hydrogen atom or a C₁₋₃ alkyl group.

Embodiment of Compound (II) includes 8-(2,6-difluorobenzyl)-5,8-dihydro-2-[4-(ethylaminocarbonylamino)phenyl]-3-(N-methyl-N-benzylaminomethyl)-5-oxoimidazo[1,2-a]pyrimidine-6-carboxylic acid ethyl ester, 8-(2,6-difluorobenzyl)-5,8-dihydro-2-[4-(methoxyaminocarbonylamino)phenyl]-3-(N-methyl-N-benzylaminomethyl)-5-oxoimidazo[1,2-a]pyrimidine-6-carboxylic acid isopropyl ester, and 8-(2,6-difluorobenzyl)-5,8-dihydro-2-[4-(ethylaminocarbonylamino)phenyl]-3-(N-methyl-N-benzylaminomethyl)-5-oxoimidazo[1,2-a]pyrimidine-6-carboxylic acid isopropyl ester.

Examples of a salt of Compound (II) are the same as those of a salt of the Compound (I) mentioned above.

Compound (II) can be prepared by a known method such as a method described in WO99/33831 or JP-A 11-315079, or the similar method.

In addition, the non-peptidic compound having gonadotropin releasing hormone antagonistic activity includes quinoline derivatives described in WO97/14682 or JP-A 9-169735, imidazopyrimidine derivatives, pyrrolopyrimidine derivatives and triazolopyrimidine derivatives described in WO01/29044, imidazopyrimidine derivatives and pyrrolopyrimidine derivatives described in WO00/69859, compounds described in WO01/55119, compounds described in WO97/44037, compounds described in WO97/44041, compounds described in WO97/44321, compounds described in WO97/44339, a 3,5,5,8,8-pentamethyl-5,6,7,8-tetrahydro-2-naphthalene derivative described in Bioorganic & Medicinal Chemistry Letters 12 (2002) 3467-3470, a 3,5,5,8,8-pentamethyl-5,6,7,8-tetrahydro-2-naphthalene derivative and 5-[(3,5,5,8,8-pentamethyl-5,6,7,8-tetrahydro-2-naphthalenyl)methyl]-N-(2,4,6-trimethoxyphenyl)-2-furamide described in Bioorganic & Medicinal Chemistry Letters 12 (2002) 3635-3639.

The toxicity of the non-peptidic compound having GnRH antagonistic activity is low.

The non-peptidic compound having GnRH antagonistic activity can be formulated into a pharmaceutical composition in various dosage forms according to a known method, and then orally or parenterally administered to a mammal (e.g. human, monkey etc.) suffering from hot flash.

In addition, the non-peptidic compound having GnRH antagonistic activity can be used for suppressing occurrence of a feminized breast.

For administration, specifically, the non-peptidic compound having GnRH antagonistic activity is mixed with a pharmaceutically acceptable carrier and then usually formulated into a solid preparation such as a tablet, a capsule, a granule or a powder to be administered orally, or an injection, a suppository or a sublingual tablet to be administered parenterally (e.g. intravenously, subcutaneously, or intramuscularly). Alternatively, the non-peptidic compound may be formulated into a sustained-release preparation such as a sublingual tablet or a microcapsule and then administered sublingually, subcutaneously or intramuscularly.

The pharmaceutically acceptable carrier includes various organic or inorganic carrier substances which are conventionally used as pharmaceutical material, and it is incorporated in a solid preparation as an excipient, a lubricant, a binder or a disintegrant; or in a liquid preparation as a solvent, a solubilizer, a suspending agent, an isotonizing agent, a buffer or a soothing agent. If necessary, pharmaceutical additives such as preservatives, antioxidants, coloring agents and sweeteners can be used.

Preferable examples of the excipient include lactose, white sugar, D-mannitol, starch, crystalline cellulose and light silicic anhydride. Preferable examples of the lubricant include magnesium stearate, calcium stearate, talc and colloidal silica. Preferable examples of the binder include crystalline cellulose, white sugar, D-mannitol, dextrin, hydroxypropylcellulose, hydroxypropylmethylcellulose and polyvinylpyrrolidone. Preferable examples of the disintegrant include starch, carboxymethylcellulose, carboxymethylcellulose calcium, croscarmellose sodium and carboxymethylstarch sodium. Preferable examples of the solvent include water for injection, alcohol, propylene glycol, macrogol, sesame oil and corn oil. Preferable examples of the solubilizer include polyethylene glycol, propylene glycol, D-mannitol, benzyl benzoate, ethanol, trisaminomethane, cholesterol, triethanolamine, sodium carbonate and sodium citrate. Preferable examples of the suspending agent include surfactants such as stearyltriethanolamine, sodium laurylsulfate, laurylaminopropionic acid, lecithin, benzalkonium chloride, benzethonium chloride and monostearic acid glycerin; hydrophilic polymers such as polyvinyl alcohol, polyvinyl pyrrolidone, carboxymethylcellulose sodium, methylcellulose, hydroxymethylcellulose, hydroxyethylcellulose and hydroxypropylcelluloce. Preferable examples of the isotonizing agent include sodium chloride, glycerin and D-mannitol. Preferable examples of the buffer include phosphate buffer, acetate buffer, carbonate buffer and citrate buffer. Preferable examples of the soothing agent include benzyl alcohol. Preferable examples of the preservative include parahydroxybenzoic acid esters, chlorobutanol, benzyl alcohol, phenethyl alcohol, dehydroacetic acid and sorbic acid. Preferable examples of the antioxidant include sulfite and ascorbic acid.

A daily dose varies depending on severity of a symptom; the age, sex and weight of a subject to be administered; a time and an interval of administration, the nature, composition and kind of a pharmaceutical preparation; the kind of an active ingredient, being not limited particularly. For example, when orally administered for treating hot flash, a daily dose is usually 0.1 to 300 mg, preferably about 1 to 300 mg, further preferably about 10 to 200 mg for an adult and is usually administered in 1 to 4 divided doses.

The content of the non-peptidic compound having GnRH antagonistic activity in the agent of the present invention is about 0.01 to 100% by weight of the total agent.

The non-peptidic compound having GnRH antagonistic activity may be used in combination with a drug for lowering the level of a sex hormone [e.g. GnRH agonist, sex hormone synthesis inhibitor (e.g. aromatase inhibitor such as anastrozole and letrozole)], a sex hormone activity inhibitor (e.g. estrogen receptor antagonist such as tamoxifen, androgen receptor antagonist such as bicalutamide), a sex hormone preparation (estrogen preparation such as premarin and raloxifene, corpus luteum hormone such as medroxyprogesterone acetate, androgen preparation such as enanthic acid testosterone, and a combinatorial agent thereof), an osteoporosis treating agent (e.g. bisphosphonic acid drug), a central drug [e.g. antianxiety, sleep-introducing agent, schizophrenia treating agent, Parkinson's treating agent, anti-dementia agent (e.g. cerebral circulation improving agent, cerebral metabolism activator etc.) etc.], a depressor, a diabetes treating agent, an anti-hyperlipemia agent, a nutrient (e.g. vitamin agent), an analgesic, a digestion absorption promoter, a gastrointestinal drug, or the like.

The non-peptidic compound having GnRH antagonistic agent may be also used in combination with an acetylcholine esterase inhibitor (e.g. tacrine, donepezil, rivastigmine, galantamine, physostigmine-DDS, ipidacrine etc.), a muscarinic acetylcholine receptor agonist, nicotinic acetylcholine receptor agonist, a Ca antagonist (e.g. nimodipine etc.), a COX-2 inhibitor (e.g. rofecoxib, celecoxib etc.), an AMPA receptor agonist, a monoamine oxidase inhibitor (e.g. selegiline-DDS), amyloid β protein secretion/aggregation inhibitor, or an Alzheimer type dementia treating drug such as nifiracetam or Memantine.

An administration method of the non-peptidic compound having GnRH antagonistic activity and a concomitant drug are not particularly limited as long as they are administered in combination. Such administration method includes (1) administration of a single preparation obtained by formulating the non-peptidic compound having GnRH antagonistic activity and a concomitant drug into a preparation, (2) simultaneous administration of two preparations obtained by formulating the non-peptidic compound having GnRH antagonistic activity and a concomitant drug into separate preparations via the same administration route, (3) administration of two preparations obtained by formulating the non-peptidic compound having GnRH antagonistic activity and a concomitant drug into separate preparations via the same administration route at different times, (4) simultaneous administration of two preparations obtained by formulating the non-peptidic compound having GnRH antagonistic activity and a concomitant drug into separate preparations via different administration routes, and (5) administration of two preparations obtained by formulating the non-peptidic compound having GnRH antagonistic activity and a concomitant drug into separate preparations via different administration routes at different times (e.g. administration in an order of non-peptidic compound having GnRH antagonistic activity and then a concomitant drug, or vice verse).

The following Reference Examples and Examples further illustrate the present invention, but do not limit the present invention.

¹H-NMR spectrum was measured with a Varian GEMINI 200 (200MHz) type spectrometer, JEOL. Ltd. LAMBDA300 (300MHz) type spectrometer or Brucca AM 500 (500MHz) type spectrometer using tetramethylsilane as an internal standard, and a total δ value is indicated in ppm. Unless otherwise indicated, "%" denotes weight percentage. A yield is denoted in mol/mol%. Other symbols used herein mean as follows:
- s:: singlet
- d:: doublet
- t:: triplet
- dt:: double triplet
- m:: multiplet
- br:: broad

Room temperature indicates, but not limited to, the range of about 15 to 25°C. Lactose, corn starch and magnesium stearate used in Examples were products meeting the specification of the Japanese Pharmacopoeia 14th Edition.

### Examples

### Reference Example 1

### 2-Amino-4-methyl-5-(4-nitrophenyl)thiophene-3-carboxylic acid ethyl ester

A mixture of 4-nitrophenylacetone (35.0 g, 195 mmol), ethyl cyanoacetate (23.8g, 195 mmol), ammonium acetate (3.1 g, 40 mmol) and acetic acid (9.1 ml, 159 mmol) was heated to reflux for 24 hours in a Dean Stark apparatus with removing produced water. After cooling, the reaction solution was concentrated under reduced pressure and the residue was partitioned between dichloromethane and aqueous sodium bicarbonate. The organic layer was washed with an aqueous sodium chloride solution, dried (MgSO₄), and the solvent was distilled off under reduced pressure. The residue was purified by silica gel column chromatography. The resulting oil was dissolved in ethanol, sulfur (5.0 g, 160 mmol) and diethylamine (16.0 ml, 160 mmol) were added, and the mixture was stirred at 60 to 70°C for 2 hours. After cooling, the reaction solution was concentrated under reduced pressure, and the residue was partitioned between dichloromethane and aqueous sodium bicarbonate. The organic layer was washed with an aqueous sodium chloride solution, dried (MgSO₄), and the solvent was distilled off under reduced pressure. The residue was purified by silica gel column chromatography and then crystallized from ether-hexane to obtain the title compound (22.2 g, 52%) as a red plate crystal.
MP: 168-170°C (recrystallization from ether-hexane)

| Elementary Analysis for C₁₄H₁₄N₂O₄S | | | |
|---|---|---|---|
| | C(%) | H(%) | N(%) |
| Calculated | 54.89; | 4.61; | 9.14 |
| Found | 54.83; | 4.90; | 9.09 |

¹H-NMR (200 MHz, CDCl₃) δ: 1.39 (3H, t, J = 7.1 Hz), 2.40 (3H, s), 4.34 (2H, q, J = 7.1 Hz), 6.27 (2H, br), 7.48 (2H, d, J = 8.7 Hz), 8.23 (2H, d, J = 8.7 Hz).
IR (KBr): 3446, 3324, 1667, 1580, 1545, 1506, 1491, 1475, 1410, 1332 cm⁻¹.

### Reference Example 2

### 5-Methyl-6-(4-nitrophenyl)-3-phenylthieno[2,3-d]pyrimidine-2,4(1H, 3H)-dione

Phenyl isocyanate (2.66 ml, 24.48 mmol) was added to a solution of the compound of Reference Example 1 (5.00 g, 16.32 mmol) in pyridine (30 ml) and the mixture was stirred at 45°C for 6 hours. The reaction solution was concentrated under reduced pressure and the resulting residue was dissolved in ethanol (6 ml). To this solution was added 28% sodium methoxide (7.86 g, 40.80 mmol), and the reaction solution was stirred at room temperature for 2 hours. Thereto 2N hydrochloric acid (25 ml, 50 mmol,) was added and the ethanol solvent was distilled off under reduced pressure. The resulting residue was filtered, washed with water-ethanol, dried under reduced pressure, and then recrystallized from ethanol to obtain the title compound (6.09 g, 98%) as a yellow powder.
mp: >300°C.

| Elementary Analysis for C₁₉H₁₃N₃O₄S·0.3H₂O | | | |
|---|---|---|---|
| | C(%) | H(%) | N(%) |
| Calculated | 59.30; | 3.56; | 10.92 |
| Found | 59.56; | 3.52; | 10.93 |

¹H-NMR (300MHz, DMSO-d₆) δ: 2.50 (3H, s), 7.31-7.46 (5H, m), 7.78 (2H, d, J = 8.8 Hz), 8.32 (2H, d, J = 8.8 Hz), 12.50 (1H, s).
IR (KBr): 1715, 1657, 1593, 1510 cm⁻¹.

### Reference Example 3

### 1-(2,6-Difluorobenzyl)-5-methyl-6-(4-nitrophenyl)-3-phenylthieno[2,3-d]pyrimidine-2,4(1H, 3H)-dione

To a solution of the compound of Reference Example 2 (52.54 g, 0.131 mol) in dimethylformamide (1.0 L) were added potassium carbonate (19.00 g, 0.138 mol), potassium iodide (22.90 g, 0.138 mol) and 2,6-difluorobenzyl chloride (22.40 g, 0.138 mol), and the mixture was stirred at room temperature for 2 hours. The reaction solution was concentrated and the residue was partitioned between chloroform and an aqueous sodium chloride solution. The aqueous layer was extracted with chloroform. The extracts were combined, washed with an aqueous sodium chloride solution and then dried (MgSO₄). The solvent was distilled off under reduced pressure. The resulting residue was purified by silica gel column chromatography to obtain the title compound (61.50 g, 93%) as a pale yellow crystal.
mp: 280-282°C.

| Elementary Analysis for C₂₆H₁₇N₃O₄SF₂ | | | |
|---|---|---|---|
| | C(%) | H(%) | N(%) |
| Calculated | 61.78; | 3.39; | 8.31 |
| Found | 61.67; | 3.46; | 8.21 |

¹H-NMR (300 MHz, CDCl₃) δ: 2.57 (3H, s), 5.38 (2H, s), 6.94 (2H, d, J = 8.1 Hz), 7.42-7.58 (8H, m), 8.29 (2H, d, J = 8.8 Hz).
IR (KBr): 1719, 1669, 1524, 1473 cm⁻¹.

### Reference Example 4

### 5-Bromomethyl-1-(2,6-difluorobenzyl)-6-(4-nitrophenyl)-3-phenylthieno[2,3-d]pyrimidine-2,4(1H, 3H)-dione

A mixture of the compound of Reference Example 3 (30.34 g, 0.060 mol), N-bromosuccinimide (12.81 g, 0.072 mol), α, α'-azobisisobutyronitrile (1.15 g, 0.007 mol) and chlorobenzene (450 ml) was stirred at 85°C for 3 hours. After cooling, the reaction solution was washed with an aqueous sodium chloride solution and dried (MgSO₄). The solvent was then distilled off under reduced pressure. The residue was recrystallized from ethyl acetate to obtain the title compound (80.21 g, 100%) as a yellow needle crystal.
mp: 228-229°C.
¹H-NMR (300 MHz, CDCl₃) δ: 4.77 (2H, s), 5.38 (2H, s), 6.96 (2H, t, J = 8.1 Hz), 7.29-7.58 (6H, m), 7.79 (2H, d, J = 8.5 Hz), 8.35 (2H, d, J = 8.5 Hz).
IR (KBr): 1721, 1680, 1524, 1473, 1348 cm⁻¹.
FAB-Mass m/z 584 (MH)⁺

### Reference Example 5

### 5-(N-benzyl-N-methylaminomethyl)-1-(2,6-difluorobenzyl)-6-(4-nitrophenyl)-3-phenylthieno[2,3-d]pyrimidine-2,4(1H, 3H)-dione

To a solution of the compound of Reference Example 4 (80.00 g, 0.119 mol) in dimethylformamide (600 ml) were added ethyldiisopropylamine (27.00 ml, 0.155 mol) and benzylmethylamine (18.45 ml, 0.143 mol) under ice-cooling. After stirred at room temperature for 2 hours, the reaction solution was concentrated and the resulting residue was partitioned between ethyl acetate and aqueous saturated sodium bicarbonate. The aqueous layer was extracted with ethyl acetate and the organic layers were combined and then dried (MgSO₄). The solvent was then distilled off under reduced pressure. The resulting residue was purified by silica gel column chromatography to obtain a yellow oil (74.90 g, 100%), which was recrystallized from ethyl acetate to obtain the title compound as a yellow needle crystal.
mp:173-174°C.

| Elementary Analysis for C₃₄H₂₆N₄O₄SF₂·0.5H₂O | | | |
|---|---|---|---|
| | C(%) | H(%) | N(%) |
| Calculated | 64.45; | 4.29; | 8.84 |
| Found | 64.50; | 4.24; | 8.82 |

¹H-NMR (300 MHz, CDCl₃) [free amine] δ: 1.31 (3H, s), 3.60 (2H, s), 3.96 (2H, s), 5.39 (2H,s), 6.95 (2H, t, J = 8.2 Hz), 7.18-7.55 (11H, m), 8.02 (2H, d, J = 9.0 Hz), 8.26 (2H, d, J = 9.0 Hz).
IR (KBr) [hydrochloride]: 1719, 1678, 1597, 1520 cm⁻¹.

### Reference Example 6

### 6-(4-Aminophenyl)-5-(N-benzyl-N-methylaminomethyl)-1-(2,6-difluorobenzyl)-3-phenylthieno[2,3-d]pyrimidine-2,4(1H, 3H)-dione

To a solution of the compound of Reference Example 5 (3.00 g, 4.80 mmol) in formic acid (30 ml) were added 1M hydrogen chloride-ether (14.4 ml, 14.4 mmol) and 10% palladium carbon powder (300 mg) under ice-cooling, and the mixture was stirred at the normal temperature and the normal pressure over 2 hours, followed by hydrogenation. The reaction solution was filtered with Celite and the filtrate was concentrated under reduced pressure. The resulting residue was partitioned between dichloromethane and aqueous saturated sodium bicarbonate. The aqueous layer was extracted with dichloromethane and the organic layers were combined and then dried (MgSO₄). The solvent was then distilled off under reduced pressure. The resulting residue was purified by silica gel column chromatography to obtain the title compound (2.41 g, 84%) as a white crystal.
mp: 205-207°C.

| Elementary Analysis for C₃₄H₂₈N₄O₂SF₂·0.1AcOEt·1.2H₂O | | | |
|---|---|---|---|
| | C(%) | H(%) | N(%) |
| Calculated | 66.09; | 5.03; | 8.96 |
| Found | 66.93; | 4.94; | 8.67 |

¹H-NMR (300 MHz, CDCl₃) δ: 2.05 (3H, s), 3.56 (2H, s), 3.83 (2H, br), 3.88 (2H, s), 5.36 (2H, s), 6.70 (2H, d, J = 8.8 Hz), 6.88-6.94 (2H, m), 7.21-7.31 (8H, m), 7.41-7.53 (5H, m).
IR (KBr): 1715, 1657, 1628, 1537 cm⁻¹.

### Reference Example 7

### 5-(N-benzyl-N-methylaminomethyl)-1-(2,6-difluorobenzyl)-6-[4-(3-methoxyureido)phenyl]-3-phenylthieno[2,3-d]pyrimidine-2,4(1H, 3H)-dione

To a solution of the compound of Reference Example 6 (5.0 g, 8.41 mmol) in dichloromethane (120 ml) was added triethylamine (2.34 ml, 16.82 mmol) under ice-cooling and the mixture was stirred. To this reaction solution was added N,N'-carbonyldiimidazole (2.73 g, 16.82 mmol) under ice-cooling. The mixture was stirred at room temperature for 42 hours. Then under ice-cooling, O-methylhydroxylamine hydrochloride (7.02 g, 84.08 mmol) and triethylamine (11.7 ml, 84.08 mmol) were added. The reaction solution was stirred for 3 hours at room temperature. The reaction solution was partitioned between chloroform and aqueous saturated sodium bicarbonate. The aqueous layer was extracted with chloroform and the extracts were combined, washed with an aqueous sodium chloride solution and then dried (MgSO₄). The solvent was distilled off under reduced pressure. The resulting residue was purified by silica gel column chromatography to obtain the pale yellow solid, which was recrystallized from chloroform-ether to obtain the title compound as a white crystal (4.52 g, 80%).
mp: 204-205°C.

| Elementary Analysis for C₃₆H₃₁N₅O₄SF₂ | | | |
|---|---|---|---|
| | C(%) | H(%) | N(%) |
| Calculated | 64.75; | 4.68; | 10.49 |
| Found | 64.61; | 4.67; | 10.31 |

¹H-NMR (300 MHz, CDCl₃) δ: 2.05 (3H, s), 3.57 (2H, s), 3.82 (3H, s), 3.90 (2H, s), 5.37 (2H, s), 6.92 (2H, d, J = 8.2 Hz), 7.16-7.31 (9H, m), 7.42-7.57 (5H, m), 7.63 (1H, s), 7.73 (2H, d, J = 8.8 Hz).
IR (KBr): 3338, 3064, 1717, 1669, 1628, 1591, 1531, 1470 cm⁻¹.

### Reference Example 8

### 5-(N-benzyl-N-methylaminomethyl)-1-(2,6-difluorobenzyl)-6-[4-(3-methoxyureido)phenyl]-3-phenylthieno[2,3-d]pyrimidine-2,4(1H, 3H)-dione hydrochloride

To a solution of the white crystal of Reference Example 7 (38.34 g, 57.42 mmol) in dichloromethane (800 ml) was added 1M etheric hydrogen chloride (100 ml) under ice-cooling, and the mixture was stirred at the same temperature for 10 minutes. The reaction solution was concentrated under reduced pressure, and the resulting residue was recrystallized from methanol-ether to obtain the title compound (40.0 g, 99%) of a white powdery crystal.
mp: 182-185°C.

| Elementary Analysis for C₃₆H₃₁N₅O₄SF₂·HCl·O.5H₂O | | | |
|---|---|---|---|
| | C(%) | H(%) | N(%) |
| Calculated | 60.63; | 4.66; | 9.82 |
| Found | 60.45; | 4.68; | 9.62 |

IR (KBr): 3440, 3042, 1713, 1665, 1628, 1593, 1539, 1473 cm⁻¹.
FAB-Mass m/z 668 (MH)⁺

### Example 1

Using the compound of Reference Example 7 (100 mg), lactose (165 mg), corn starch (25 mg), polyvinyl alcohol (4 mg) and magnesium stearate (1 mg), a tablet is produced according to a conventional method.

### Experimental Example 1

### (1) Preparation of ¹²⁵I-leuprorelin

Then microliters of a 3×10⁻⁴ M aqueous leuprorelin solution and 10 µl of 0.01 mg/ml lactoperoxidase were put into a tube and thereto 10 µl (37 MBq) of a Na¹²⁵I solution was added. The mixture was stirred, and 10 µl of 0.001% H₂O₂ was added, followed by reaction at room temperature for 20 minutes. The reaction was stopped by addition of 700 µl of a 0.05% TFA solution and the reaction mixture was purified by reverse phase HPLC. Conditions of HPLC are shown below. ¹²⁵I-leuprorelin was eluted at a retention time of 26 to 27 minutes.
Column: TSKgel ODS-80™ (TM indicates registered trademark; the same hereinafter)
CTR (4.6 mm × 10 cm) eluent:
Solvent A (0.05% TFA)
Solvent B (40% CH₃CN-0.05% TFA)
0 minutes (100% Solvent A) - 3 minutes (100% Solvent A) - 7 minutes (50% Solvent A+50% Solvent B) - 40 minutes (100% Solvent B)
Elution temperature: room temperature
Elution rate: 1 ml/min

### (2) Preparation of anterior pituitary membrane fraction containing rat GnRH receptor

Anterior pituitaries were removed from 40 Whister rats (8 weeks old, male) and washed with an ice-cooled homogenate buffer ({25 mM Tris [tris(hydroxymethyl)aminomethane]-HCl}, 0.3M saccharose, 1 mm EGTA (glycol ether diaminetetraacetic acid), 0.25 mM PMSF (phenylmethylsulfonyl fluoride), 10 U/ml aprotinin, 1 µg/ml pepstatin, 20 µg/ml leupeptin, 100 µg/ml fosforamidon, 0.03% sodium azide, pH 7.6). The pituitary glands were floated on 2 ml of a homogenate buffer and homogenized using a Polytron homogenizer. After centrifugation at 700×g for 15 minutes, the supernatant was put in a supercentrifuge tube and centrifuged at 100,000×g for 1 hour to obtain precipitates of a membrane fraction. To the precipitates was added 2 ml of an assay buffer (25 mM Tris-HCl, 1 mm EDTA (ethylenediaminetetraacetic acid) (0.1% BSA (bovine serum albumin), 0.25 mM PMSF, 1 µg/ml pepstatin, 20 µg/ml leupeptin, 100 µg/ml fosforamidon, 0.03% sodium azide, pH 7.5) to suspend the precipitates therein, which was centrifuged at 100,000×g for 1 hour. A membrane fraction was collected as precipitates, suspended again in 10 ml of the assay buffer, dispensed, and then stored at -80°C, which was thawed before use.

### (3) Preparation of CHO (Chinese hamster ovary) cell membrane fraction containing human GnRH receptor

Human GnRH receptor-expressing CHO cells (10⁹ cells) were suspended in a phosphate-buffered physiological saline (PBS-EDTA) containing 5 mM EDTA and then centrifuged at 100×g for 5 minutes. To the pellet of cells was added 10 ml of a cell homogenate buffer (10 mm NaHCO₃, 5 mM EDTA, pH 7.5), and this was homogenized using a Polytron homogenizer. After centrifugation at 400×g for 15 minutes, the supernatant was put in a supercentrifuge tube and centrifuged at 100,000×g for an hour to obtain precipitates of a membrane fraction. The precipitates were suspended in 2 ml of the assay buffer and centrifuged at 100,000×g for an hour. A membrane fraction was collected as precipitates, suspended again in 20 ml of the assay buffer, dispensed, and stored at -80°C, which was thawed before use.

### (4) Measurement of ¹²⁵I-leuprorelin binding inhibiting rate

The rat and human membrane fractions prepared in the above (2) and (3) were diluted with the assay buffer to 200 µg/ml each, 188 µl of which was put into each tube. When the anterior pituitary membrane fraction was used, 2 µl of a 0.1 mM solution of a compound in 60% DMSO (dimethyl sulfoxide) and 10 µl of 38 nM ¹²⁵I-leuprorelin were added at the same time. When the human GnRH receptor-expressing CHO cell membrane fraction was used, 2 µl of a 2 mM solution of a compound in 60% DMSO and 10 µl of 38 nM ¹²⁵I-leuprorelin were added at the same time. In order to measure a maximum binding amount, a reaction solution to which 2 µl of 60% DMSO and 10 µl of 38 nM ¹²⁵I-leuprorelin had been added was prepared. In addition, in order to measure a non-specific binding amount, a reaction solution to which 2 µl of 100 µM leuprorelin solution in 60% DMSO and 10 µl of 38 nM ¹²⁵I-leuprorelin had been added was prepared.

When the anterior pituitary membrane fraction was used, a reaction was performed at 4°C for 90 minutes and, when the human GnRH receptor-expressing CHO cell membrane fraction was used, a reaction was performed at 25°C for 60 minutes. After the reaction, the reaction solution was suction-filtered using a polyethyleneimine-treated Whatmann glass filter (GF-F). After the filtration, radioactivity of ¹²⁵I-leuprorelin remaining on the filer was measured using a γ-counter.

(TB-SB)/(TB-NSB)×100 (SB: radioactivity when compound is added, TB: maximum binding radioactivity, NSB: non-specific binding radioactivity) was calculated to obtain a binding inhibiting rate (%) of each test substance. In addition, by varying the concentration of a test substance, an inhibiting rate was obtained, and the concentration of a test substance which inhibits the binding by 50% (IC₅₀ value) was calculated from the Hill plot. Results are shown below:

**[Table 1]**

| Test substance | Binding inhibiting rate (%) Rat (1 µM) | IC₅₀ value (µM) Human |
|---|---|---|
| Compound of Reference Example 8 | 27 | 0.0001 |

### Experimental Example 2

To 32 premenopausal healthy females (20 years old to 45 years old), 1 to 25 mg/day of the compound of Reference Example 7 was administered for 14 days. As a result, the serum estradiol concentration was reduced, but hot flash was not observed.

### Industrial Applicability

A preventing or treating agent for hot flash which comprises a non-peptidic compound having gonadotropin releasing hormone antagonistic activity of the present invention is low toxic and has excellent hot flash preventing or treating effect.

## Claims

1. A preventing or treating agent for hot flash which comprises a non-peptidic compound having gonadotropin releasing hormone antagonistic activity.

2. The agent according to claim 1, wherein the compound is a compound capable of entering the brain.

3. The agent according to claim 1, wherein the compound is a fused heterocyclic compound.

4. The agent according to claim 1, wherein the compound is a compound represented by the formula: 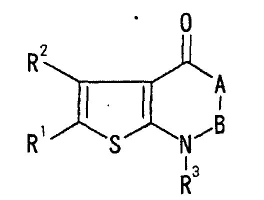 wherein R¹ represents (1) a hydrogen atom, (2) a group linking via a carbon atom, (3) a group linking via a nitrogen atom, (4) a group linking via an oxygen atom or (5) a group linking via a sulfur atom,
R² represents (1) a hydrogen atom, (2) a group linking via a carbon atom, (3) a group linking via a nitrogen atom, (4) a group linking via an oxygen atom or (5) a group linking via a sulfur atom,
R³ represents (1) a hydrogen atom, (2) alkyl or (3) -(CH₂)ₚQ (wherein p represents an integer of 0 to 3 and Q represents an optionally substituted homocyclic group or an optionally substituted heterocyclic group), 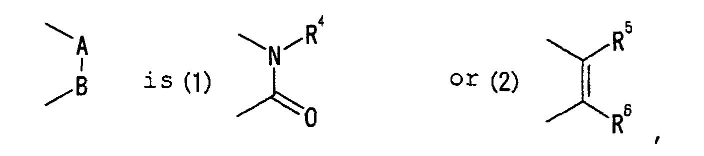 R⁴ represents (1) a hydrogen atom, (2) alkyl optionally substituted with alkoxy, (3) optionally substituted aryl, (4) optionally substituted aralkyl or (5) optionally substituted cycloalkyl,
R⁵ represents (1) a hydrogen atom, (2) formyl, (3) cyano, (4) C₁₋₆alkyl optionally substituted with (i) a group linking via a sulfur atom or (ii) a group linking via an oxygen atom, (5) an optionally substituted heterocyclic group, (6) a group linking via a nitrogen atom, (7) a group linking via an oxygen atom, (8) a group linking via a sulfur atom, (9) optionally esterified, thioesterified or amidated carboxyl or (10) -C(O)R⁷ (wherein R⁷ represents an optionally substituted hydrocarbon group), and
R⁶ represents (1) a hydrogen atom or (2) a group linking via a carbon atom, or a salt or prodrug thereof.

5. The agent according to claim 4, wherein R¹ is optionally substituted C₆₋₁₄ aryl, R² is (1) C₁₋₃alkyl substituted with a group linking via a nitrogen atom or (2) a group linking via a nitrogen atom, R³ is -(CH₂)ₚQ (wherein p represents an integer of 0 to 3 and Q represents an optionally substituted homocyclic group or an optionally substituted heterocyclic group), 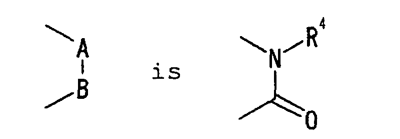 R⁴ is (1) C₁₋₆alkyl optionally substituted with C₁₋₆alkoxy or (2) optionally substituted C₆₋₁₄aryl.

6. The agent according to claim 1, wherein the compound is a compound represented by the formula: 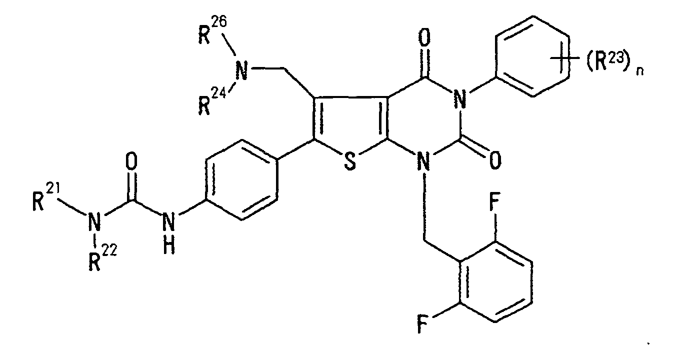 wherein R²¹ and R²² each represent (1) a hydrogen atom (2) hydroxy (3) C₁₋₄alkoxy, (4) C₁₋₄alkoxy-carbonyl or (5) optionally substituted C₁₋₄alkyl, R²³ represents (1) a hydrogen atom, (2) halogen, (3) hydroxy or (4) optionally substituted C₁₋₄alkoxy, or two R²³ adjacent to each other may be linked to form C₁₋₄ alkylenedioxy, R²⁴ represents (1) a hydrogen atom or (2) C₁₋₄alkyl, and R²⁶ represents (1) optionally substituted C₁₋₄alkyl or (2) a group represented by the formula: 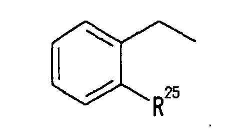 wherein R²⁵ represents a hydrogen atom or may be taken together with R²⁴ to form a heterocycle, and n represents an integer of 0 to 5, or a salt thereof.

7. A method for preventing or treating hot flash, which comprises administering an effective amount of a non-peptidic compound having gonadotropin releasing hormone antagonistic activity to a mammal.

8. Use of a non-peptidic compound having gonadotropin releasing hormone antagonistic activity for preparation of a preventing or treating agent for hot flash.
